# EUROPEAN PATENT SPECIFICATION

(11) **EP 1 879 618 B1**
(45) Date of publication and mention of the grant of the patent: **04.10.2017**
(21) Application number: 06770164.9
(22) Date of filing: 10.05.2006
(51) Int. Cl.: A61K 38/19, A61K 38/20, A61K 38/21

(54) **A METHOD FOR MODULATING HLA CLASS II TUMOR CELL SURFACE EXPRESSION WITH A CYTOKINE MIXTURE**
VERFAHREN ZUR MODULATION DER OBERFLÄCHENEXPRESSION EINES HLA-TUMORS DER KLASSE II MIT EINER CYTOKIN-MISCHUNG
PROCEDE DE MODULATION DE L'EXPRESSION DU HLA DE CLASSE II SUR LA SURFACE D'UNE CELLULE TUMORALE AU MOYEN D'UN MELANGE DE CYTOKINES

(30) Priority: 10.05.2005 US 125176
(43) Date of publication of application: 23.01.2008
(73) Proprietor: Cel-Sci Corporation, Vienna, VA 22182 (US); Talor, Eyal, Baltimore MD 21209 (US)
(72) Inventor: TALOR, Eyal, Baltimore, MD 21209 (US)
(74) Representative: Price, Phillip
(86) International application number: PCT/US2006/018055
(87) International publication number: WO 2006/122178

(56) References cited:
- WO-A-2005/007086
- WO-A-2005/120550
- TÍMÁR JÓZSEF ET AL: "Neoadjuvant immunotherapy of oral squamous cell carcinoma modulates intratumoral CD4/CD8 ratio and tumor microenvironment: a multicenter phase II clinical trial." JOURNAL OF CLINICAL ONCOLOGY : OFFICIAL JOURNAL OF THE AMERICAN SOCIETY OF CLINICAL ONCOLOGY 20 MAY 2005, vol. 23, no. 15, 20 May 2005 (2005-05-20), pages 3421-3432, XP002552350 ISSN: 0732-183X
- HARRIS D T ET AL: "IMMUNOLOGIC APPROACHES TO THE TREATMENT OF PROSTATE CANCER" SEMINARS IN ONCOLOGY, W.B. SAUNDERS, vol. 26, no. 4, 1 January 1999 (1999-01-01), pages 439-447, XP000910090 ISSN: 0093-7754
- TIMAR J ET AL: "THE EFFECT OF LEUKOCYTE INTERLEUKIN INJECTION (MULTIKINE) TREATMENT ON THE PERITUMORAL AND INTRATUMORAL SUBPOPULATION OF MONONUCLEAR CELLS AND ON TUMOR EPITHELIA: A POSSIBLE NEW APPROACH TO AUGMENTING SENSITIVITY TO RADIATION THERAPY AND CHEMOTHERAPY IN" LARYNGOSCOPE, TRIOLOGICAL FOUNDATION, ST LOUIS, US, vol. 113, 1 December 2003 (2003-12-01), pages 2206-2217, XP008048121 ISSN: 0023-852X
- FEINMESSER M. ET AL.: 'Histologic and Immunohistochemical Characterization of Tumor and Inflammatory Infiltrates in Oral Squamous Cell Carcinomas Treated with Local Multikine Immunotherapy: the Macrophage at the Front Line' EUR. ARCH. OTORHINOLARYNNGOL vol. 261, no. 7, August 2004, pages 359 - 368, XP003009564
- Talor et al.: Journal of Clinical Oncology, 2004 ASCO Annual Meeting Proceedings (Post-Meeting Edition)., vol. 22, no. 14S 1 July 2004 (2004-07-01), page abstract No. 2605, Retrieved from the Internet: URL:Journal of Clinical Oncology, 2004 ASCO Annual Meeting Proceedings (Post-Meeting Edition).Vol 22, No 14S (July 15 Supplement), 2004: 2605 [retrieved on 2014-03-06]

## Description

### INTRODUCTION

This invention relates to a method for altering the composition of tumor infiltrating mononuclear cells, increasing CD4+/CD8+ ratio, increasing tumor stroma/epithelial ratio and modulating HLA (Human Leukocyte Antigen) class II expression on a tumor cell surface with a serum-free and mitogen-free mixture having specific cytokine ratios from the group IL -1β, TNF-α, IFN-γ, GM-CSF to IL-2, respectively. The serum-free mitogen-free mixtures comprised of cytokine ratios include Leukocyte Interleukin (LI) or Multikine®.

### BACKGROUND OF THE INVENTION

Mechanisms of immunological escape are a central concern in tumor immunology. Immunogenic neoplasms appear to elude the effector arm of the immune system through antigenic modulation, antigen masking, as well as the production of blocking factors, genetic factors or by the presence or absence of tumor products or growth factors.

One determinant of immunologic escape may be HLA class 11 expression on tumor cells. HLA class II molecules play an important role in the process of antigen presentation and recognition by CD4+T-lymphocytes. Their expression may reflect the presence or absence of an important co-factor necessary for tumor elimination by host immunocompetent cells. HLA class II expression is found on the target issues of the majority of human autoimmune diseases as well as certain cancers and activated IL-2, receptor-expressing T lymphocytes. The
observation that the local site of autoimmune diseases and certain types of cancer results in over-expressed HLA class II suggests that tissue antigen-presenting capacity contributes significantly to the mechanism of autoimmune disease perpetuation or cancer progression by continually reactivating auto-antigen-reactive T lymphocytes.

Autoimmune conditions such as Graves' thyroiditis seem to reactivate auto-antigen-reactive T lymphocytes wherein interferon-gamma (IFN-γ) induces class II expression on thyrocytes (Feldman et al., "Molecular mechanisms involved in human autoimmune diseases: relevance of chronic antigen presentation Class II expression and cytokine production", Immunol Suppl, 2: 66-73 (1989)). The thyrocytes then express class II antigens and present their auto-antigens to T-cells cloned from the thyroid tissue of Graves' disease patients.

In cancer, immunological escape appears to occur when the balance between factors for tumor growth and destruction favor the tumor. Factors that may contribute to immunological escape include tumor kinetics, antigenic modulation, antigen masking and blocking factors. For example, antigenic modulation facilitates escape by removing the target antigens that the immune system's effector cells would recognize. This is known to occur when administering xenogeneic antibodies during immunotherapy. Tumor escape from effector cells may also occur because certain molecules bind to the surfaces of the tumor cell and mask the tumor antigens thereby preventing adhesion of attacking lymphocytes.

Tumor products other than antigens can also cause a subversion of the immune response. Such products include prostaglandins and other humoral factors that act to impair inflammatory responses, chemotaxis, and complement cascade. The ability of a tumor to escape from immunological control may depend on a balance between the effectiveness of the immune system and a variety of factors that promote escape.

With regard to the major histocompatibility locus, it is noted that molecular changes in the expression of HLA class I antigen and other molecules such as HLA-DR, CD44 and ICAM-1 (intracellular adhesion molecule-1) may affect tumor progression. Interestingly, the presence or absence of certain cell surface antigens is associated with a poor prognosis for certain types of cancer. For example, several groups have reported that HLA-class I antigen deletion in the tumor cell surface relates to a more aggressive behavior of bladder cancer (Klein B et al., "HLA class I antigen expression in solid tumors", Isr J Med Sci 32: 1238-1243 (1996); Nouri AM et al., "Induction of MHC antigens by tumor cell lines in response to interferons", Eur J Cancer 28(A): 1110-1115 (1992); Nouri et al., "Defective expression of adhesion molecules on human bladder tumour and human tumour cell lines", Urol Int 56: 6-12 (1996); Nouri A et al., "Selective and non selective loss of immunoregulatory molecules (HLA-A,B,C antigens and LFA-3) in transitional cell carcinoma", Br J Cancer 62: 603-606 (1990)). Data from other studies indicate that the lack of expression of MHC class I antigens or co-stimulatory molecules on the target (tumor) cells, FasL expression, or signaling defects can also contribute to impaired immune response to oral squamous cell carcinomas (Cruz et al., "Lack of MHC class I surface expression on neoplastic cells and poor activation of the secretory pathway of cytotoxic cells in oral squamous cell carcinomas", Br J Cancer 81:881-889 (1999); Lang et al., "Impairment of T-cell activation in head and neck cancer in situ and in vitro", Arch Otolaryngol Head Neck Surg 125:82-88 (1999); Hoffmann et al., "Spontaneous apoptosis of circulating T lymphocytes in patients with head and neck cancer and its clinical importance", Clin Cancer Res 8:2553-2562 (2002); Kuss et al., "Effector CD8+CD45RO-CD27-T cells have signaling defects in patients with squamous cell carcinoma of the head and neck", Br J Cancer 88:223-230 (2003)).

Major Histocompatibility Complex (MHC) class II molecules also appear to affect tumor progression. MHC gene products can be grouped into two classes. Class I products are found on all nucleated cells while class II products are found on cells of the immune system. HLA-DR, a class II MHC molecule, is involved in the processing of antigenic signals and is expressed on the surface of squamous cell carcinomas from the head and neck. Major histocompatibility complex (MHC) class II molecules may play a role in generating immunological signals and modulating the quantity of these immunologically active antigens.

Antigens on the cell surface can also change when normal cells turn into cancer cells. These new or altered antigens flag down to the detriment of the host body, immune defenders, including cytotoxic T cells, natural killer cells, and macrophages. Conversely, tumor cells can avoid surveillance by expressing certain molecules.

Another possible mechanism for immune escape is cell anergy by failure to express a co-stimulatory signal. Full activation of CD4⁺ T-cells appears to require two signals. The absence of a co-stimulatory signal results in cell anergy when a first signal is received through the T-cell receptor (TCR). This situation may arise where a malignant cell presents a MHC Class II antigen thereby triggering cell anergy in the absence of a necessary co-stimulatory signal resulting in proliferative non-responsiveness by CD4⁺ T-cells.

Normally, the sequence of events in T-cell activation has three distinct stages of cell-cell interaction between antigen presenting cells (APCs) and antigen specific T-cells to induce an antigen specific immune response. First, adhesion occurs when APCs and T-cells randomly interact both in the circulation and lymphoid tissues via cell surface ligands and adhesion receptors. Second, recognition occurs if APCs can process, transport, and present a sufficient quantity of the specific peptide antigen in the context of the MHC. Antigen-MHC (or in human HLA) complexes will then be recognized by T-cells via the T-cell receptor (TCR). Endogenous peptides derived from intracellular proteins are generally presented with MHC class I (in human HLA-A, B, or C) whereas exogenously processed peptide antigens derived from circulation proteins are generally presented with MHC class II (in human HLA-DR, DP, or DQ). Third, costimulation occurs after T-cells have been activated via binding of the TCR providing T-cells with an additional signal that enhances activation.

Interleukin-2 which is produced by activated T lymphocytes stimulates T-cell, B-cell, and NK-cell proliferation and induces lymphokine production. Interleukin-2 secretion by helper T-cells responding to MHC II associated antigens appear to provide the major stimulus for activation of cytotoxic T-cells and NK-cells directed against tumor cells. For example, lymphoid cells incubated with IL-2 are capable of lysing tumor cells. These activated cells are known as lymphokine-activated killer cells.

Interleukin-2 has been administered systematically in a number of phase I clinical trials. A pilot clinical trial of Interleukin-2 was reported in patients with recurrent inoperable squamous cell carcinoma of the head and neck (De Stefani et al., "Treatment of oral cavity and oropharynx squamous cell carcinoma with perilymphatic interleukin-2: clinical and pathologic correlations", J Immunother 19:125-133 (1966)). Of the five cases evaluated, partial or complete resolution of tumor was noted in four patients while no responses were noted in patients who had received a previous functional or radical neck dissection. It was theorized that this was due to the removal of draining lymph nodes and the loss of any local lymphoid response. Many pathological conditions such as infections, cancer, autoimmune disorders *etc.,* are characterized by the inappropriate expression of certain molecules wherein these molecules serve as "markers" for a particular pathological or abnormal condition. This evidence supports the characterization of head and neck cancer as an immunosuppressive disease wherein immune reactivity is maximally suppressed in tumor infiltrating lymphocytes (TIL), followed by proximal lymph node lymphocytes (LNL), distal LNL and peripheral blood lymphocytes (PBL).

The PBL count correlates with cellular immunity and seems to have prognostic implications for patients with carcinoma of the head and neck. The most complex assays of the cell-mediated immune system are those that monitor lymphocyte reactivity. The tests are *in vitro* assays and expose the patient's lymphocytes to a variety of stimuli. The stimuli may consist of common recall antigens, specific tumor antigens, or nonspecific stimulants resulting in a depression in the total number of T lymphocytes and hence, an absolute decrease in T- and B-cell levels in patients with head and neck cancer. In addition, the absolute T-cell counts decreases with advancing disease. A decrease in the stimulation of lymphocytes in patients with head and neck cancer also occurs.

Intratumoral CD4⁺/CD8⁺ ratios also seem to affect carcinomas of the head and neck. CD4⁺ molecules found on T helper cells recognize and bind class II HLA markers found on dendritic cells, monocytes, macrophages, and B-cells and transmit the signal for the T helper cells to secrete more lymphokines such as IL-2. CD8⁺ molecules, found on T killer cells recognize and bind class I HLA markers and transmit the signal for the T killer cell to secrete proteins such as perforin that punch holes in the membrane of the foreign cell and directly cause its lysis and death.

A successful immune response to oral squamous cell carcinoma may require the reversal of the inherently low intratumoral CD4⁺/CD8⁺ ratio. Notably, the predominance of CD8⁺ over CD4⁺ cells in solid tumors is not specific for oral squamous cell carcinomas. Similar low CD4⁺/CD8⁺ ratios have been reported in basal cell carcinoma, cervical, and breast cancers >Rohrbach et al., "Immunology and growth characteristics of ocular basal cell carcinoma", Graefes Arch Clin Exp Ophthalmol 239:35-40 (2001); Santin et al., "Tumor-infiltrating lymphocytes contain higher numbers of type 1 cytokine expressors and DR+ T cells compared with lymphocytes from tumor draining lymph nodes and peripheral blood in patients with cancer of the uterine cervix", Gynecol Oncol 81:424-432 (2001); Murta et al., "Lymphocyte subpopulations in patients with advanced breast cancer submitted to neoadjuvant chemotherapy", Tumori 86:403-407 (2000)).

On the other hand, high percentages of CD4⁺ T-cells or high CD4⁺/CD8⁺ ratios are correlated with a better prognosis or response to immunotherapy of melanoma, B-cell non-Hodgkin's lymphoma, renal cell carcinoma, and cervical carcinoma (Håkansson et al., "Tumour-infiltrating lymphocytes in malignant melanoma and response to interferon alpha treatment", Br J Cancer 74:670-676 (1996); Ansell et al., "CD4+ T-cell immune response to large B-cell non-Hodgkin's lymphoma predicts patient outcome", J Clin Oncol 19:720-726 (2001); Igarashi et al., "Effect of tumor-infiltrating lymphocyte subsets on prognosis and susceptibility to interferon therapy in patients with renal cell carcinoma", Urol Int 69:51-56, (2002); Sheu et al., "Reversed CD4/CD8 ratios of tumor-infiltrating lymphocytes are correlated with the progression of human cervical carcinoma", Cancer 86:15371543, (1999)).

CD4⁺ T-cells also appear to play a central role in initiating and maintaining antitumor immunity by providing help for CD8⁺ CTL by stimulating antigen presenting cells and sustaining immunological memory or in some cases by performing effector functions via cytokine secretion or direct cytolysis (Goedegebuure et al., "The role of CD4+ tumor-infiltrating lymphocytes in human solid tumors", Immunol Res 14:119-131 (1995); Hung et al., "The central role of CD4+ T cells in the antitumor immune response", J Exp Med 188:2357-2368 (1998); Gao et al., "Antigen-specific CD4+ T-cell help is required to activate a memory CD8+ T cell to a fully functional tumor killer cell", Cancer Res 62:6438-6441 (2002)).

With regard to regional immune reactivity in head and neck cancer, Berlinger *et al.* evaluated the morphologic pattern of lymph nodes from 84 patients with head and neck cancer and correlated their findings with survival wherein patients whose lymph nodes demonstrated an active immunologic response had a greater survival time (Berlinger et al., "Immunobiology Otolaryngology 3rd ed.", Philadelphia: Harcourt Brace Jovanovich, Inc; 741-45 (1991)). On the other hand, patients whose lymph nodes had a depleted or unstimulated pattern did not survive 5 years. The results are clearly indicative of a relationship between regional immunoreactivity and survival in head and neck malignancy. This finding may be attributable to tumor cells expressing certain surface antigens from exposure to certain cytokines.

Most studies of systemic immunity in patients with head and neck malignancies have yielded evidence for decreased immunologic function. Whether these changes are due to the predisposing conditions or are a function of the malignancy itself is not known. Although immunotherapy is considered a relatively specific approach to cancer treatment in which activation of immunological effector mechanisms are used to destroy cancer cells, there are several feasible immune strategies to achieve these goals. Among them is the use of cytokines to augment anticancer immune effector mechanisms (Tímár et al., "Molecular pathology of tumor metastasis III. Target array and combinatorial therapies", Pathol Oncol Res 9:49-72 (2003); Whiteside TL, "Immunobiological and immunotherapy of head and neck cancer", Curr Oncol Rep 3:46-55 (2001)).

Another feasible immune strategy uses cytokines to augment anticancer immune effector mechanisms with local IL-2 treatment introduced as a neoadjuvant immunobiotherapy prior to the surgical resection of OSCC. Some therapeutic interventions use recombinant human IL-2 (rhIL-2), while others use natural leukocyte-derived IL-2 preparation in different tumor types including head and neck cancer (Barrera et al., "Combination immunotherapy of squamous cell carcinoma of the head and neck", Arch Otolaryngol Head Neck Surg 126:345-351 (2000); Cortesina et al. "Interleukin-2 injected around tumor-draining lymph nodes in head and neck cancer", Head Neck 13:125-131 (1991); De Stefani et al. "Treatment of oral cavity and oropharynx squamous cell carcinoma with perilymphatic interleukin-2: clinical and pathologic correlations", J Immunother 19:125-133 (1996); Rivoltini et al., "In vivo interleukin 2-induced activation of lymphokine-activated killer cells and tumor cytotoxic T-cells in cervical lymph nodes of patients with head and neck tumors", Cancer Res 50:5551-5557 (1990)).

However, the data shows highly variable response rates for local rhIL-2 administrations (6-65%) (Vlock et al., "Phase Ib trial of the effect of peritumoral and intranodal injections of interleukin-2 in patients with advanced squamous cell carcinoma of the head and neck: an eastern cooperative oncology group trial", J Immunother 15:134-139 (1994)). Certain phase III trials involving the local administration of rhIL-2 to OSCC patients resulted in a significant increase in disease-free survival as well as increased overall survival (De Stefani et al., "Improved survival with perilymphatic interleukin-2 in patients with resectable squamous cell carcinoma of the oral cavity and oropharynx", Cancer 95:90-97 (2002)). Histological examination of the tumor tissue obtained from rhIL-2-treated head and neck cancer patients revealed an enrichment in T-cell subsets paralleled by focal necrosis and occasional eosinophilic infiltration (Whiteside et al., "Evidence for local and systemic activation of immune cells by peritumoral injections of interleukin 2 in patients with advanced squamous cell carcinoma of the head and neck", Cancer Res 53:5654-5662 (1993); De Stefani et al. "Treatment of oral cavity and oropharynx squamous cell carcinoma with perilymphatic interleukin-2: clinical and pathologic correlations", J Immunother 19:125-133 (1996); Valente et al., "Infiltrating leukocyte populations and T-lymphocyte subsets in head and neck squamous cell carcinoma from patients receiving perilymphatic injections of recombinant interleukin 2", Mod Pathol 3:702-708 (1990)).

Other phase II clinical trials with a mixture of naturally occurring cytokines including natural IL-2 exhibited response rates of 30% to 50% (Barrera et al., "Combination immunotherapy of squamous cell carcinoma of the head and neck", Arch Otolaryngol Head Neck Surg 126:345-351 (2000); Hadden et al., "A trial of IRX-2 in patients with squamous cell carcinomas of the head and neck", Int Immunopharmacol 8:1073-1081, (2003); Meneses et al., "Histologic findings in patients with head and neck squamous cell carcinoma receiving perilymphatic natural cytokine mixture (IRX-2) prior to surgery", Arch Pathol Lab Med 122:447-454 (1998); Verastegui et al., "A natural cytokine mixture (IRX-2) and interference with immune suppression induce immune mobilization and regression of head and neck cancer", Int J Immunopharmacol 19:619-627 (1997)).

Histopathological analysis indicated that this natural cytokine mixture (containing IL-2) induced predominantly T-lymphocytic infiltration of the tumor tissue and caused tumor fragmentation depending on the dose. Still other phase II trial with lower doses of LI (Leukocyte Interleukin, Injection, LI, also known as Multikine®), showed similar response rates and induced CD3⁺/CD25⁺ T-lymphocytic infiltration of tumor cell nests and entry of the cancer cells into cell cycle (Tímár *et al.,* "The effect of Leukocyte Interleukin, Injection (Multikine) treatment on the peritumoral and intratumoral subpopulation of mononuclear cells and on tumor epithelia: a possible new approach to augmenting sensitivity to radiation therapy and chemotherapy in oral cancer - a multicenter phase I/II clinical trial", *Laryngoscope* 113:2206-2217 (2003)).

Local treatment with a mixture of natural cytokines (including IL-2) is another feasible approach to the treatment of OSCC.

Therefore, there is a need for determining and modulating the relationship in the cellular infiltrate of the tumor particularly as they relate to tumor infiltrating mononuclear cells, increasing CD4+/CD8+ ratio, increasing tumor stroma/epithelial ratio and modulating HLA class II expression on a tumor cell surface with a naturally derived serum-free and mitogen-free mixture containing or inducing desirable affects on immunocompetence.

There is also a need for a neoimmunoadjuvant treatment prior to conventional therapy (surgery followed by radiotherapy) of OSCC in conjunction with such methods and the proper dosages resulting in safe and efficacious administration of such therapies.

### SUMMARY OF THE INVENTION

The present invention is based on methods for altering the composition of tumor infiltrating mononuclear cells, increasing CD4+/CD8+ ratio, increasing a tumor stroma/epithelial ratio and modulating HLA class II expression on a tumor cell surface with a serum-free and mitogen-free Leukocyte Interleukin, Injection (LI) mixture or Multikine® comprised of specific ratios of cytokines IL-1β to IL-2, TNG-α to IL-2, IFN-γ to IL-2 and GM-CSF to IL-2. More specifically, the invention relates to their use in preparing a medicament to treat cancer. In particular, as disclosed in claim 1.

The methods described herein, namely for altering the composition of tumor infiltrating mononuclear cells, increasing CD4+/CD8+ ratio, increasing tumor stroma/epithelial ratio and modulating HLA class II expression on a tumor cell surface relate to a serum-free and mitogen free cytokine mixture being administered three times a week over a two week period in a range from about 20 IU to 12000 IU wherein IU represent International Units for Interleukin-2 given in World Health Organization 1^{st} International Standard for Human 11-2, 86/504. In another embodiment, the serum-free and mitogen-free cytokine mixture is administered five times a week over a three week period in a range from about 20 IU to 12000 IU wherein IU represent International Units for Interleukin-2 given in World Health Organization 1^{st} International Standard for Human IL-2, 86/504.

Specific applications of the methods herein described include administering peritumorally the total daily dose of Leukocyte Interleukin, Injection (LI) or Multikine® five (5) times per week for three (3) weeks. The total daily dose can be 200, 400, 800, 1200, 1600, 2400, 3200, 4800, 8000,
9600 and 12000IU/mL as IL-2 wherein IU represents International Units for Interleukin-2 given in World Health Organization 1^{st} International Standard for Human IL-2, 86/504.

Specific applications disclosed herein include administering half a total daily dose of peritumorally of a Leukocyte Interleukin, Injection (LI) or Multikine® and administering the other half of the total daily dose perlymphatically five (5) times per week for three (3) weeks. The total daily dose can be 200, 400, 800, 1200, 1600, 2400, 3200, 4800, 8000, 9600 and 12000 IU/mL as IL-2 wherein IU represents International Units for Interleukin-2 given in World Health Organization 1^{st} International Standard for Human IL-2, 86/504. Half the total dose is the peritumoral dose wherein the peritumoral dose is injected into at least four different sites around the tumor mass. Therefore, if 800 IU/mL is the total daily dose, 400 IU is injected peritumorally into four different sites around the tumor mass wherein one quarter (1/4) of the peritumoral dose is injected into each of the four sites such that each site receives approximately 100 IU/mL as IL-2. LI is administered intra-dermally at the circumferential margins of the visible/palpable tumor mass. However, it will be understood that the methods will not be limited to four sites wherein four or less or more than four sites are contemplated by the invention. The remaining half of 400 IU of the exemplary total dose of 800 IU as IL-2 is sequentially administered perilymphatically ipsilateral to the tumor site on the same visit. The perilymphatic injections are administered at the posterior mandibular area in the area of the jugular lymphatic chain ipsilateral to the injected tumor mass.

There is also disclosed Leukocyte Interleukin, Injection (LI) having specific ratios of cytokine to interleukin 2 (IL-2) as follows: IL-1β to IL-2 at a ratio range of 0.4 -1.5, and preferably at 0.7+/-0.1 (IL-1β/IL-2), TNF-α to IL-2 at a ratio range of 3.2-11.3, and preferably at 9.5+/1.8 (TNF-α/IL-2), IFN-γ at a ration range of 1.5-10.9, and preferably at 4.0+/-0.5 (GM-CSF/IL-2).

In specific applications described herein, the serum-free and mitogen-free cytokine preparation or pharmaceutical composition has further different cytokines and other small biologically active molecules wherein the ratio of each of the small biologically active molecules to IL-2 is as follows: IL-3 to IL-2 in a ratio range of 0.38-0.68, preferably at 0.53+/-0.15, IL-6 to IL-2 in a ratio range of 37.2-53.8, preferably at 46+/-5.9, IL-8 to IL-2 in a ratio range of 261-561.5, preferably at 411+/-10.6. IL-1α to IL-2 in a ratio range of 0.56-0.94, preferably at 0.75+/-0.19, IL-10 to IL-2 in a ratio range of 2.82-3.22, preferably at 3.0+/- 0.18, IL-16 to IL-2 in a ratio range of 1.16-2.84, preferably at 1.84+/0.68, G-CSF to IL-2 in a ratio range of 2.16-3.78, preferably at 2.97+/-0.81, TNF-β to IL-2 in ratio range of 1.17 -2.43, preferably at 1.8+/-0.63, MIP-1α to IL-2 in a ratio range of 15.7-37.16, preferably at 22.7+/-7.0, MIP-1β to IL-2 in a ratio range of 17.1-28.5, preferably at 22.8+/- 5.7, a RANTES to IL-2 in a ratio range of 2.3 - 2.7, preferably at 2.5+/- 0.13, a EGF to Il-2 in a ratio range of 0.267 - 0.283, preferably at 0.275+/- 0.008, PGE₂ to IL-2 in a ratio range of 3.63- 5.42, preferably at 4.5+/- 0.87 and TxB₂ to IL-2 in a ratio range of 23.47 - 25.13, preferably at 24.3+/- 0.83.IL-12 is present in only trace quantities is defined at 100 pg/mL or less.

Also herein described is a method for modulating HLA Class II tumor cell surface expression, which comprises administering to a patient in need thereof a serum-free and mitogen-free cytokine mixture comprised of specific ratios of cytokines selected from the group IL-1β, TNF-α, IFN-γ, GM-CSF, Interleukin-2 (IL-2) where the specific ratios of IL-1β, TNF-α, IFN-γ, GM-CSF to IL-2 are: IL-2 at a ratio range of 0.4 - 1.5; TNF-α to IL-2 at a ratio range of 3.2 - 11.3; IFN-γ to IL-2 at a ratio range of 1.5 -10.9; and GM-CSF to IL-2 at a ratio range of 2.2 - 4.8.

There is also described a method for increasing CD4+/CD8+ ratio, which comprises administering to a patient in need thereof a serum-free and mitogen-free cytokine mixture comprised of specific ratios of cytokines selected from the group IL-1β, TNF-α, IFN-γ, GM-CSF, Interleukin-2 (IL-2) where the specific ratios of IL-1β, TNF-α, IFN-γ, GM-CSF to IL-2 are: IL-1β to IL-2 at a ratio range of 0.4 -1.5; TNF-α to IL-2 at a ratio range of 3.2 - 11.3; IFN-γ to IL-2 at a ratio range of 1.5 -10.9; and GM-CSF to IL-2 at a ratio range of 2.2 - 4.8.

Herein described is a method for altering the composition of tumor infiltrating mononuclear cells, which comprises administering to a patient in need thereof a serum-free and mitogen-free cytokine mixture comprised of specific ratios of cytokines selected from the group of IL-1β, TNF-α, IFN-γ, GM-CSF, Interleukin-2 (IL-2_ where the specific ratios of IL-1β, TNF-α to IL-2 at a ratio range of 3.2 - 11.3; IFN-γ to IL-2 at a ratio range of 0.4 -1.5; TNF-α to IL-2 at a ratio range of 3.2 -11.3; IFN-γ to IL-2 at a ratio range of 1.5 -10.9; and GM-CSF to IL-2 at a ratio range of 2.2 -4.8.

Also herein described is a method is provided for increasing a tumor stroma/epithelial ratio, which comprises administering to a patient in need thereof a serum-free and mitogen-free cytokine mixture comprised of specific ratios of cytokines selected from the group of Il-1β, TNF-α, IFN-γ, GM-CSF, Interleukin-2 (IL-2) where the specific ratios of Il-1β, TNF-α, IFN-γ, GM-CSF to IL-2 are: IL-1β to IL-2 at a ratio range of 0.4-1.5; TNF-α- to IL-2 at a ratio range
of 3.2 -11.3; IFN-γ to IL-2 at a ratio range of 1.5- 10.9, and GM-CSF to IL-2 at a ratio range of 2.2- 4.8.

### BRIEF DESCRIPTION OF THE DRAWINGS

The invention will now be explained in greater detail by the following description and specific embodiments and with the aid of the accompanying drawings:

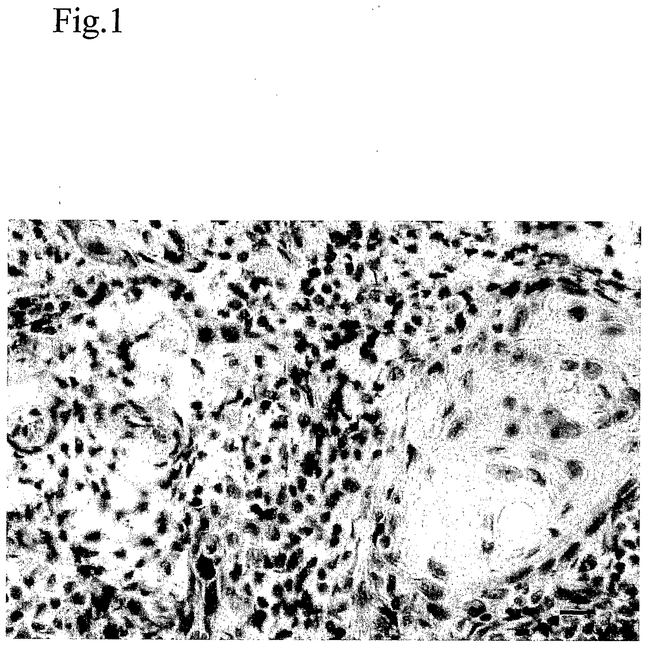

Fig. 1 is an immuno-histochemistry CD4+ staining of a tumor in oral squamous cell carcinoma (OSCC) following pre-treatment with Leukocyte Interleukin (LI) Injection wherein the bar = 100µM.

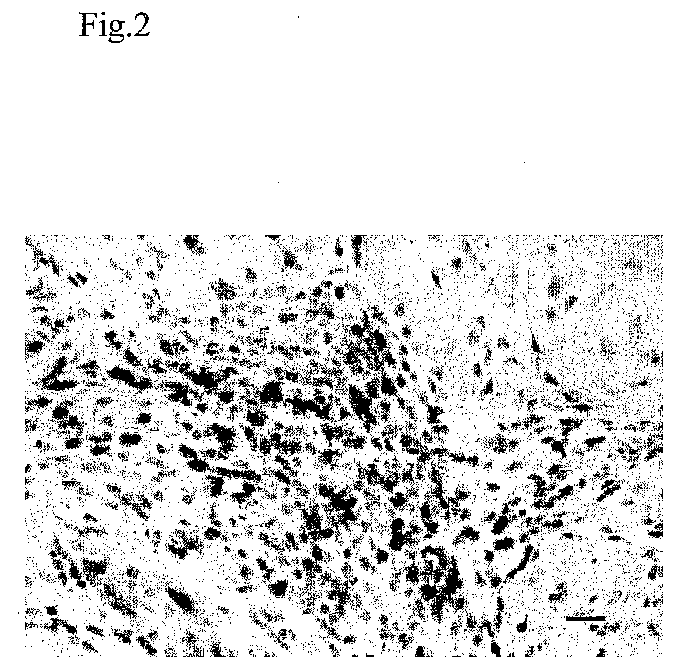

Fig.2 is an immuno-histochemistry CD8+ staining of T-cells in a control sample of OSCC stroma wherein the bar =100µM.

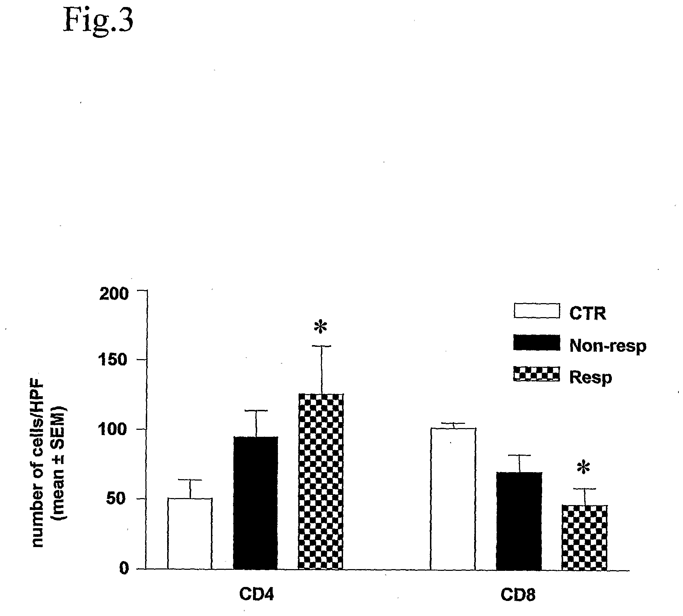

Fig.3 is a graph showing the change in stroma density of CD4+ and CD8+ T-cells where CTR= control, untreated (n=20), Non-resp + LI treated non-responder (n = 11), Resp -= LI-treated responder^{b} (n = 6) and data are Mean ± SEM, *=p<0.05.
Fig.4 is a graph showing the change intraepithelial density of CD4₊ and CD8₊ T-cells where CTR = control, untreated (n=20), Non-resp +LI-treated non responder^{a} (n=11), Resp= LI treated responder^{b} (n=6) and date are Mean± SEM, * =p<0.05.
Fig. 5 is a graph showing the CD4⁺/CD8⁺ ratio where CTR = control, untreated (n=20), Non-resp = LI-treated non-responder^{a} (n=11), Resp = LI-treated responder^{b} (n=6) and data are Mean ± SEM, *= p<0.05.

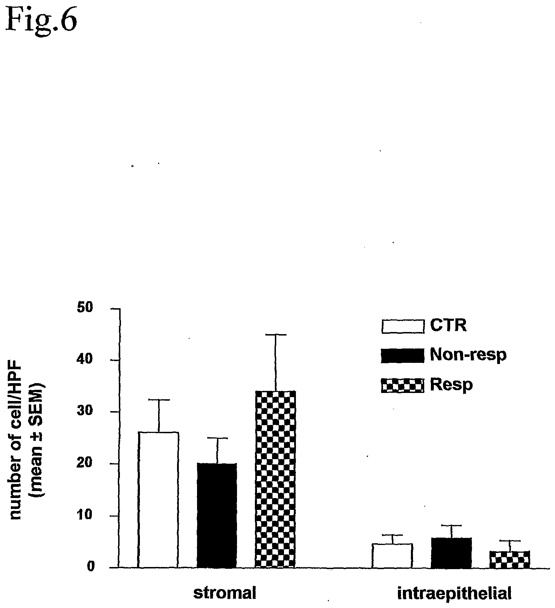

Fig. 6 is a graph showing the stromal and intraepithelial density of CD25/IL-2R⁺ OSCC cells where CTR = control, untreated (n=20), Non-resp = LI-treated non-responder^{a} (n=11), Resp = LI-treated responder^{b} (n=6) and data are Mean ± SEM, *= p<0.05.

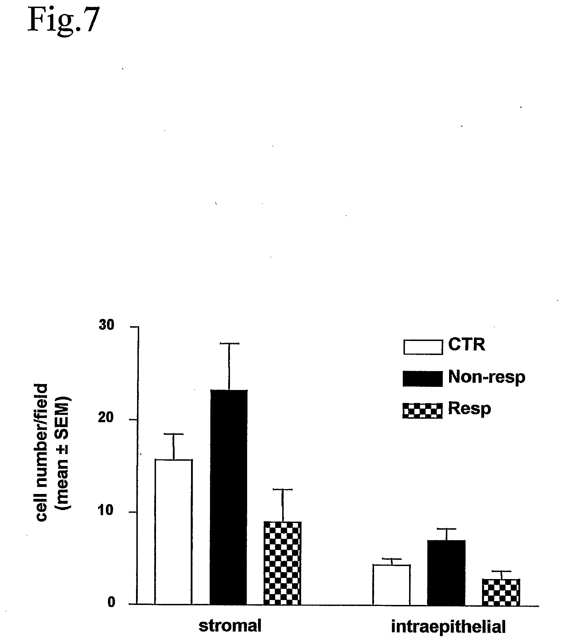

Fig. 7 is a graph showing the stromal and intraepithelial density of OX40⁺ cells in OSCC cells where CTR = control, untreated (n=20), Non-resp = LI-treated non-responder^{a} (n=11), Resp = LI-treated responder^{b} (n=6) and data are Mean ± SEM, *= p<0.05.
Fig. 8 is a graph showing the stromal and intraepithelial density of CD45R0⁺ cells in OSCC where CTR= control, untreated group (n=20), Non-resp= LI-treated non-responder^{a} (n=11), Resp= LI-treated responder^{b} (n=6). Data are means± SEM. The observed alterations in the LI-treated groups are statistically not significant.
Fig. 9 is a graph showing the density of dendritic cells in OSCC samples detected by CD1a immunoreaction where tumor zone: R1= surface zone, R2= tumor center, R3= tumor-stroma interface where CTR= control, untreated group (n=20), Non-resp= LI-treated non-responder^{a} (n=11), Resp= LI-treated responder^{b} (n=6). Data are means± SEM, *= p<0.05.
Fig. 10 is a graph showing the stromal and intraepithelial density of macrophages in OSCC samples detected by CD68 immunoreaction where CTR= control, untreated group (n=20), Non-resp= LI-treated non-responder^{a} (n=11), Resp= LI-treated responder^{b} (n=6). Data are means± SEM, *= p<0.05.

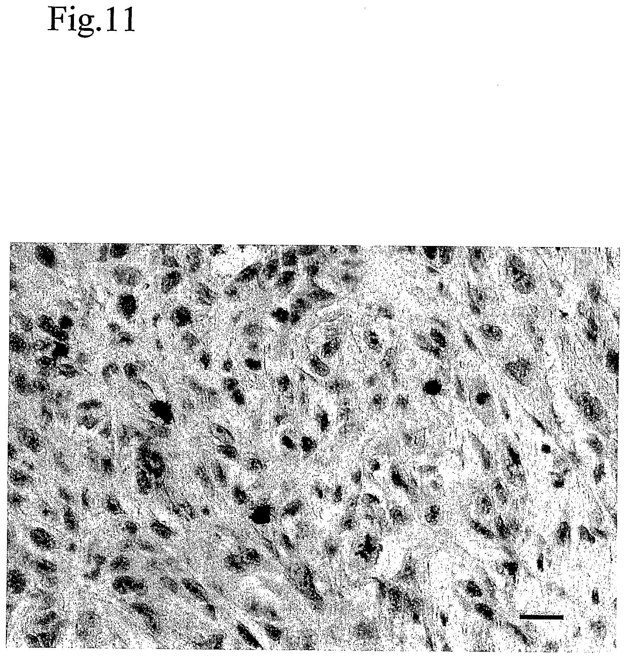

Fig. 11 is an immuno-histochemistry staining showing a control sample for the localization of neutrophils intraepithelially using myeloperoxidase immunohistochemistry (red signal) where the bar = 100 µm.

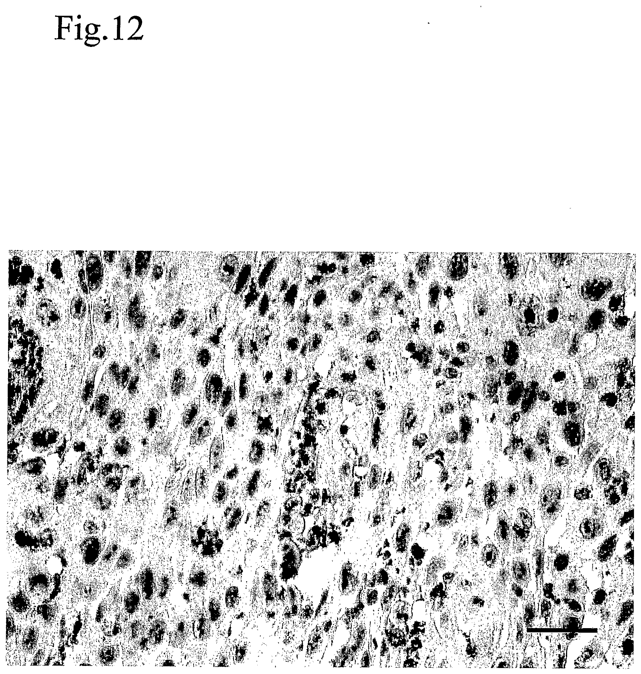

Fig. 12 is an immuno-histochemistry staining of a LI-treated sample for the localization of neutrophils intraepithelially using myeloperoxidase immunohistochemistry (red signal) where the bar = 100 µm.
Fig. 13 is a graph showing the density of neutrophils in OSCC samples where CTR= control, untreated group (n=20), Non-resp = LI-treated non-responder^{a} (n=11), and Resp = LI-treated responder^{b} (n=6). Data are Mean ± SEM, *= p<0.05.
Fig. 14 is a color microscopic photograph of H&E stained paraffin sections of an OSCC control sample showing that the OSCC control sample is devoid of any necrotic event where the bar = 500 µm.
Fig. 15 is a color microscopic photograph of H&E stained paraffin sections of an OSCC, LI treated sample showing induced microscopic epithelial necrosis where the bar = 500 µm.

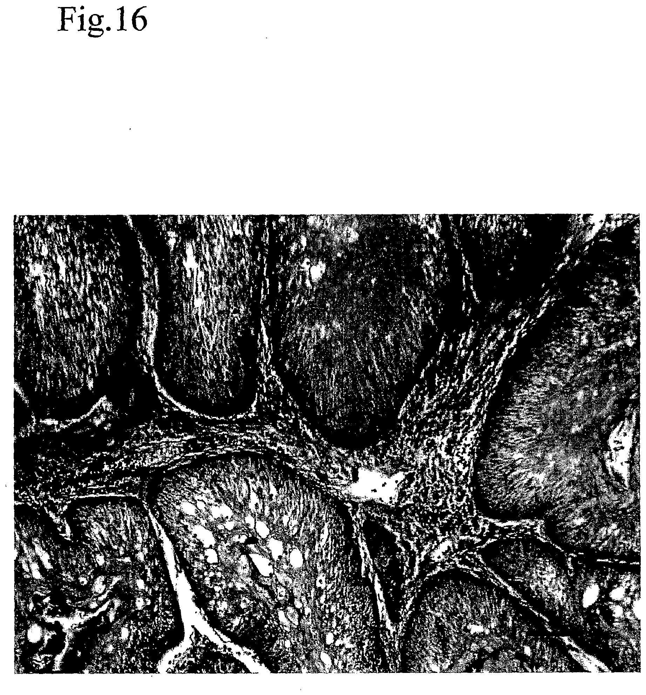

Fig. 16 is a color microscopic photograph of collagenous stroma in OSCC of an untreated control showing a peritumoral fibrous collagen rim. The bar = 200 µm and the tumor stroma is indicated in the blue area.

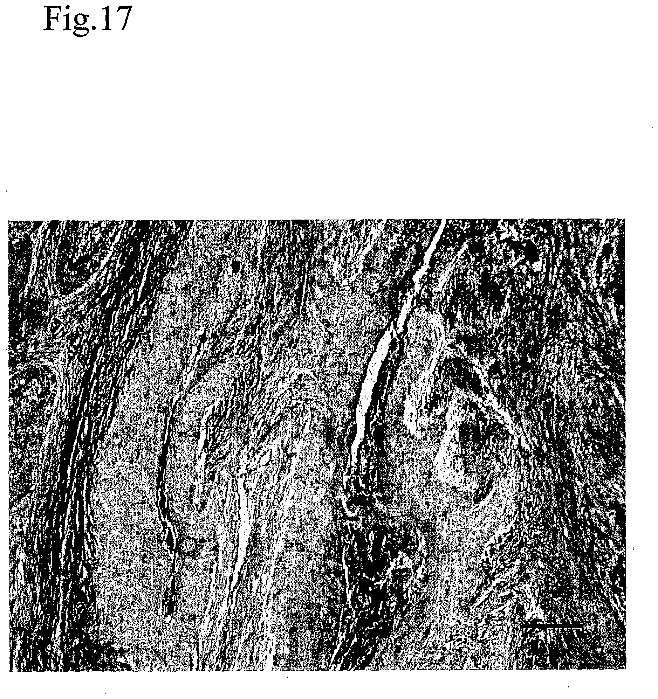

Fig. 17 is a color microscopic photograph of collagenous stroma in OSCC of an LI-treated OSCC showing accumulation of interstitial collagen fibers between cancer cell nests. The bar = 200 µm and the tumor stroma is indicated in the blue area.
Fig. 18 is a graph showing the morphometry of collagenous stroma of OSCC in percent area of stroma where CTR= control, untreated (n=20), Non-resp= LI-treated non-responder^{a} (n=11), and Resp= LI- treated responder^{b} (n=6). Data are means ± SEM, *p=<0.05
   ^{a}LI-treated non-responder = LI-treated advanced and oral squamous cell carcinoma patients having a physical response to treatment of <30% in comparison to base-line tumor volume (area) reduction at the longest poles of the palpable tumor and at the widest point perpendicular to the longest direction as measured by a physical examination.
   ^{b}LI-treated responder= LI-treated advanced oral squamous cell carcinoma patients having a >50% tumor volume (area) reduction as measured in^{a}.

### DETAILED DESCRIPTION

The present invention is based on methods for altering the composition of tumor infiltrating mononuclear cells, increasing CD4₊ /CD8₊ ratio, increasing a tumor stroma/epithelial ratio and modulating HLA class II expression on a tumor cell surface with a serum-free and mitogen-free cytokine mixture comprised of specific ratios of IL-1β to IL-2, TNF-α, INF-γ to IL-2 and GM-CSF to IL-2. More specifically, the invention relates to their use in preparing a medicament to treat cancer, in particular as disclosed in claim 1. One such cytokine mixture is Leukocyte Interleukin, Injection (LI), or Multikine® which has demonstrated immuno-modulatory capabilities. The clinical significance of the immuno-suppression is cancer patients unexpectedly impacts the composition of tumor infiltrating mononuclear cells, a CD4₊/CD8₊ ratio, a tumor stroma/epithelial ratio and HLA class II expression on a tumor cell surface. In particular, administering Leukocyte Interleukin, Injection (LI) doses of 200, 400, 800, 1200, 1600, 2400, 3200, 4800, 8000, 9600 and 12000 IU/mL peritumorally three(3) times a week for two(weeks) or five(5) times per week for (3) weeks alters the composition of
tumor infiltrating mononuclear cells, increases CD4+/CD8+ ratio, increases a tumor stroma/epithelial ratio and modulates HLA class II expression on a tumor cell surface in patients initially treated with LI for head and neck cancer.

The peritumoral dose during cancer treatment can be performed by injecting 100 IU/mL as IL-2 into four different sites around a tumor mass for an exemplary 800 IU/mL total dose for which 400 IU/mL is injected peritumorally wherein each of the four sites are injected with one quarter (1/4) of the peritumoral dose. The remaining half of the total dose is then administered perilymphatically ipsilateral to the tumor site sequentially and on the same visit. The perilymphatic injections are given at one site at the posterior mandibular area in the area of the jugular lymphatic chain ipsilateral to the injected tumor mass.

### Definitions

IL-2- Interleukin 2 (IL-2): A 15.5-kD glycoprotein synthesized by CD4+ helper T-lymphocytes (Formally known as T cell Growth Factor). IL-2 has an autocrine effect acting on the CD4+ T-lymphocytes that produce it and on other cells of the immune system (including B-lymphocytes, CD8+ T-lymphocytes, NK [Natural Killer] cells and others).

IL-1β - Interleukin 1 beta (IL-1β): A 17-kD cytokine synthesized by activated mononuclear phagocytes is found in free form in the circulation and mediates inflammatory responses. It acts on CD4+ T lymphocytes to help facilitate their proliferation, and acts on B-lymphocytes as a growth and differentiation factor. It also induces the synthesis of IL-6 by mononuclear phagocytes.

TNF-α - Tumor Necrosis Factor alpha (TNF-α): A 157 amino acid (aa) residues protein, synthesized by stimulated monocytes, macrophages, B lymphocytes, T lymphocytes, an NK cells among others, found in a trimmeric form in the circulation. TNF mediates direct anti-tumor action, causing tumor cell lysis, facilitates leukocyte recruitment, inducing angiogenesis and promotes fibroblast proliferation.

IFN-γ- Interferon Gamma (IFN-γ): A 21-24-kD glycoprotein homodimer synthesized by activated T lymphocytes and NK cells, is a powerful activator of monocytes increasing monocytes ability to destroy intracellular microorganisms and tumor cells. It has direct anti-viral and anti-proliferative activity, and causes many cell types to express Class II MHC (Major Histocompatibility Complex) cell surface molecular complex, as well as increasing the expression of Class I MHC.

GM-CSF - Granulocyte Macrophage - Colony Stimulating Factor (GM-CSF): A 127 aa protein found as a monomer in the circulation, produced by macrophages and T lymphocytes, fibroblast and endothelial cells. It is a growth factor for hemopoietic cells, and stimulates the growth and differentiation of myelomonocytic lineage.

IL-3 - Interleukin - 3 (IL-3): A 20-kD lymphokine synthesized by activated CD4+ T helper lymphocytes, acts as a colony-stimulating factor by facilitating the proliferation of some hematopoietic cells and promoting the proliferation and differentiation of T lymphocytes.

IL-6 - Interleukin - 6 (IL-6): A 26-kD cytokine produced by activated T lymphocytes, mononuclear phagocytes, endothelial cells, and fibroblasts. It acts on many cells but has a special function in enabling activated B-lymphocytes to differentiate into antibody secreting plasma cells, and induces hepatocytes to form acute-phase proteins (implicated in inflammatory responses) as well as fibrinogen.

IL-8 - Interleukin - 8 (IL-8): An 8-kD protein produced by macrophages and endothelial cells. Is a powerful chemotactic factor for neutrophils and T lymphocytes, and facilitates neutrophil adherence to endothelial cells.

IL-1α - Interleukin 1 alpha (IL-1α): A 17-kD cytokine (like IL-1β) is cleaved from a 33-kD precursor molecule, synthesized by activated mononuclear phagocytes, is rarely found in free form in the circulation and acts as a membrane-associated substance. It assists IL-1β in mediating inflammatory responses.

IL-10 - hiterleukin - 10 (IL-10): An 18-kD polypeptide produced by CD4+ and CD 8+ T lymphocytes, monocytes, macrophages, activated B lymphocytes, and keratinocytes. It inhibits macrophages ability to present antigen particularly to T_{H}1-type cells, and secrete IL-6 and TNF.

IL-16 - Interleukin - 16 (IL-16): A 14-kD tetrameric protein produced by CD8+ T lymphocytes, eosinophils, mast cells and respiratory epithelial cells. It has strong chemoattraction properties for CD4+ T lymphocytes and monocytes.

G-CSF - Granulocyte Colony Stimulating Factor (G-CSF): A 22 - 25-kD homodimer glycoprotein produced by macrophages, endothelial cells, fibroblasts and stromal cells. It increases granulocyte progenitor cells in the marrow, and sustains increase in blood neutrophils. It also enhances the ability of neutrophils to exhibit enhanced super-oxide production thought to be important in the destruction of microbially infected cells and tumor cells.

TNF-β - Tumor Necrosis Factor beta (TNF-β): A 25-kD protein produced by activated lymphocytes. It can kill tumor cells in culture, and stimulates proliferation of fibroblasts. In addition it mimics most of the other actions of TNF-α.

MIP-1α - Macrophage Inflammatory Protein - 1 alpha (MIP-1α): A 66-aa monomeric protein produced by macrophages and other cells. It is a chemo-attractant for monocytes, T lymphocytes and eosinophils.

RANTES - An 8-kD protein produced by T lymphocytes and is a chemo-attractant to monocytes, T lymphocytes and eosinophils, and promotes inflammation.

EGF - Epidermal Growth Factor (EGF): A trisulfated polypeptide of 53-aa residues. EGF is a member of the tyrosin kinase family, and has multiple functions including stimulation of the mitogenic response and assisting in wound healing.

PGE₂ - Prostaglandin E₂ (PGE₂): PGE₂ belong to a family of biologically active lipids derived from arachidonic acid through the cyclooxygenase enzymatic reaction. It is released by activated monocytes and blocks MHC Class II expression on T lymphocytes and macrophages.

TxB₂ - Thromboxane B₂ (TxB₂): TxB₂ is a member of biologically active compounds derived from polyunsaturated fatty acids by isomerization of prostaglandin and endoperoxidase PGH₂ via the enzyme thromboxane synthetase. TxB₂ has a physiological role in thromboembolic disease, and anaphylactic reactions.

CD25⁺ Cells - CD25 is a single chain glycoprotein, often referred to as the α-chain of the Interleukin-2-receptor (IL-2R) or the Tac-antigen, that has a mol wt of 55 kDa and is present on activated T- and B-cells and activated macrophages. It functions as a receptor for IL-2. Together with the β-chain of the IL-2R, the CD25 antigen forms a high-affinity receptor complex for IL-2.

HLA-DR⁺ Lymphocytes - Lymphocytes containing human leukocyte antigen (HLA)-DR antigens, a group of polymorphic glycoproteins determined by a glue sequence found in a leukocyte loci located on chromosome 6, the major histocompatibility loci in humans.

IU (International Units) - A unit of measure of the potency of biological preparations by comparison to an international reference standard of a specific weight and strength *e*.*g*., WHO 1^{st} International Standard for Human IL-2, 86/504. International Units are the only recognized and standardized method to report biological activity units that are published and are derived from an international collaborative research effort.

U (Units as a measure of biological activity) - Shorthand for a variety of named "units", which each laboratory derives as a reference, which is further unique to the laboratory where the work is being performed. Each "unit" is different from one laboratory to another laboratory and is not a globally recognized standard such as International Units (IU) .

USP - U.S. Pharmacopeia Monographs.

*P* - "p < 0.01": A term in mathematical statistics that denotes the level of probability of an event occurring under pre-set conditions.

ANOVA (Analysis of Variances) - A single factor analysis as described in Statistics and mathematical textbooks e.g., "Handbook of Statistical Methods for Engineers and Scientists", Harrison M. Wadsworth, Jr., Ed., McGraw Hill 1990, and "Statistical Operations Analysis of Health Research Data", Robert P. Hirsch and Richard K. Riegelman, Eds. Blackwell Science Inc., 1996.

Chi Square (χ²) distribution - A sample variance analysis generated by considering the sum of squares of independent standard normal random variables where the squares of independent standard normal random variables are summed together resulting in a chi square (χ²) distribution with n degrees of freedom.

### Leukocyte Interleukin, Injection (LI)

In the manufacturing process, mononuclear cells are separated from human donor "buffy coats" by step-gradient centrifugation and cultured with PHA to enhance production and secretion of IL-2 and other cytokines from the donor white blood cells in culture as disclosed in U.S. Patents 5,093,479, 4,390,623, 4,388,309, 4,406,830, 4,661,447, 4,681,844 and 4,464,355, all of which are incorporated herein by reference. Subsequently, the culture supernatant is aseptically harvested, clarified and subjected to a commercial virus exclusion process. The supernatant is then further concentrated approximately 10 fold by ultrafiltration and microfiltration.

At this point, Human Serum Albumin, Inj. USP is added and the concentrate is then buffered to a physiological pH and brought to a target IL-2 concentration per the label claim (example 400 IU/mL). The concentrate is then subjected to a second micro-filtration (0.22 micron-rated filter) and aseptically dispensed into sterile serum-type vials and labeled by its IL-2 content. Product potency is measured by the incorporation of radio-labeled thymidine by a cytotoxic T-lymphoid line (CTLL-2). The final injectable agent is further tested by ELISA for the presence of five marker cytokines: IL-2, IL-1β, GM-CSF, IFN-γ, and TNF-α.

Leukocyte Interleukin, Injection (LI) or Multikine® is provided frozen in a borosilicate glass serum vial containing 2.2 mL of drug at the label claim as IL-2 (400 IU/mL) for peritumoral, intratumoral, perilymphatic or subcutaneous administration. Leukocyte Interleukin, Injection (LI) or Multikine® is subjected to quality control tests for identity, sterility, bacterial endotoxins, pH, and total protein concentration. Each vial is inspected for particulate contamination and appearance. The preparation has a total protein content of 3 mg/mL wherein the material is supplied sterile and pyrogen free. Leukocyte Interleukin, Injection (LI) or Multikine® has an assigned expiration date of 24 months from date of manufacture when the drug is stored at -20°C.

There are three "families" of cytokines in LI that together may be important to the unique biological activity of LI. They may include direct cytotoxic/cytostatic and virocidal/virostatic cytokines such as TNF-α, and IFN-γ, lympho-proliferative cytokines such as IL-1, and IL-2 and chemotactic cytokines such as IL-6, IL-8 and MIP-1α. However, it is expressly noted that this theory of the invention or any other theory expressed in this patent in no way limits the scope of the claims.

The different cytokine and small biological molecules that constitute LI are all derived from the lectin (PHA) *in vitro* stimulation of human peripheral blood mononuclear cells that include T-cells, B-cells, and macrophages. Centrifugation on a Ficoll-Paque gradient separates the white blood cells (including T-cells, B-cells, and macrophages) from donor whole blood, and a series of washes (in physiologically buffered media) facilitates the isolation of lymphocytes, and the removal of red blood cells, cellular debris and other unwanted cellular components from the isolated white cell component of the whole donor blood.

Leukocyte Interleukin, Injection (LI) or Multikine® is tested using a characterization protocol and does not contain the following cytokines and other small biologically active molecules: IL-4, IL-7, and IL-15, TfR, sICAM, PDGF-AB, IFN-α, EPO, LTC 4, TGF-β2, FGF basic, Angiogenin, sE-selectin, SCF, and LIF. LI contains only trace quantities (just above the level of detection of the assay of IL-12, and LTB4.

Leukocyte Interleukin (LI) or Multikine® may further be used as a component of an immunomodulatory composition together with one or more pharmaceutically acceptable carriers or adjuvants, either prophylactically or therapeutically. When provided for use prophylactically, the immunomodulatory composition is provided in advance of any evidence of infection or disease. While it is possible for LI to be administered in a pure or substantially pure form, a pharmaceutical composition, formulation or preparation may also be used.

The formulations described herein, both for clinical and for human use, comprise LI as described above together with one or more pharmaceutically acceptable carriers and, optionally, other therapeutic ingredients, especially therapeutic immunological adjuvants. The carrier(s) must be "acceptable" in the sense of being compatible with the other ingredients, of the formulation and not deleterious to the recipient thereof.

In general, the formulations are prepared by uniformly and intimately bringing into association the active ingredient with liquid carriers or finely divided solid carriers or both, and then, if necessary, bringing the product into the desired formulation. The term "pharmaceutically acceptable carrier" as used herein refers to any carrier, diluent, excipient, suspending agent, lubricating agent, adjuvant, vehicle, delivery system, emulsifier, disintegrant, absorbent, preservative, surfactant, colorant, flavorant, or sweetener. The formulations may conveniently be presented in unit dosage form and may be prepared by any method well-known in the pharmaceutical art.

Formulations suitable for intravenous, intramuscular, subcutaneous, or intraperitoneal
nasal, etc. administration conveniently comprise sterile aqueous solutions of the active ingredient(s) with solutions which are preferably isotonic with the blood of the recipient. The compounds of the present disclosure may also be administered orally, parenterally, by inhalation spray, topically, rectally, buccally, vaginally, or via an implanted reservoir in dosage formulations containing conventional non-toxic pharmaceutically-acceptable carriers, adjuvants and vehicles. The term parenteral as used herein includes subcutaneous, intravenous, intramuscular, intraperitoneally, intrathecally, intraventricularly, intrasternal and intracranial injection or infusion techniques.

Such formulations may be conveniently prepared by dissolving solid active ingredients in water containing physiologically compatible substances such as sodium chloride (e.g. 0.1 -2.0M), glycine, and the like, and having a buffered pH compatible with physiological conditions to produce an aqueous solution and rendering the solution sterile. These may be present in unit or multi-dose containers, for example, sealed ampules or vials.

The compounds disclosed herein may also be administered in the form of sterile injectable preparations, for example, as sterile injectable aqueous or oleaginous suspensions. These suspensions may be formulated according to techniques known in the art using suitable dispersing or wetting agents and suspending agents. The sterile injectable preparations may also be sterile injectable solutions or suspensions in non-toxic parentally-acceptable diluents or solvents, for example, as solutions in 1, 3-butanediol. Among the acceptable vehicles and solvents that may be employed are water, Ringer's solution and isotonic sodium chloride solution. In addition, sterile, fixed oils are conventionally employed as solvents or suspending mediums. For this purpose, any bland fixed oil may be employed including synthetic mono
-di-glycerides. Fatty acids such as oleic acid and its glyceride derivatives, including olive oil and castor oil, especially in their polyoxyethylated versions are useful in preparation of injectables. These oil solutions or suspensions may also contain long-chain alcohol diluents or dispersants.

The compounds may also be administered topically, especially when the conditions addressed for treatment involve areas or organs readily accessible by topical application including disorders of the eye, the skin, or the lower intestinal tract. Suitable topical formulations are readily prepared for each of these areas.

For topical application to the eye, or ophthalmic use, the compounds can be formulated as micronized suspensions in isotonic, pH adjusted sterile saline, or preferably, as solutions in isotonic, pH adjusted sterile saline, either with or without a preservative such as benzylalkonium chloride. Alternatively, the ophthalmic uses of Multikine® may be formulated in an ointment such a petroleum.

For topical application to the skin, the compounds can be formulated in suitable ointment containing the compound suspended or dissolved in, for example, a mixture with one or more of the following: mineral oil, liquid petroleum, white petroleum, propylene glycol, polyoxyethylene compound, emulsifying wax and water. Alternatively, the compounds can be formulated in a suitable lotion or cream containing the active compound suspended or dissolved in, for example, a mixture of one or more of the following: mineral oil, sorbitan monosterate, Polysorbate 60, cetyl esters wax, cetearyl alcohol, 2-octldodecanol, benzyl alcohol and water.

The amount of active ingredient that may be combined with the carrier materials to produce a single dosage form will vary depending upon the host treated and the particular mode of administration. Some factors include the activity of the specific compound employed, the age, body weight, general health, sex, and diet of the patients; the time of administration, rate of excretion, drug combination, and the severity of the particular disease being treated and form of administration.

Pharmaceutical methods may also be employed to control the duration of action. Controlled release preparations may be achieved through the use of polymer to complex or absorb the peptide. The controlled delivery may be exercised by selecting appropriate macromolecules (for example, polyester, polyamino acids, polyvinyl, pyrrolidone, ethylenevinylacetate, methylcellulose, carboxymethylcellulose, or protamine sulfate) and the concentration of macromolecules as well as the methods of incorporation in order to control release.

For example, Leukocyte Interleukin, Injection (LI) or Multikine® may be incorporated into a hydrophobic polymer matrix for controlled-release over a period of days. Such controlled-release films are well known to the art. Particularly preferred are transdermal delivery systems. Other examples of polymers commonly employed for this purpose that may be used in the present invention include non-degradable ethylene-vinyl acetate copolymer and degradable lactic acid-glycolic acid copolymers which may be used externally or internally. Certain hydrogels such as poly(hydroxyethylmethacrylate) or poly(vinylalcohol) also may be useful, but for shorter release cycles then the other polymer releases systems, such as those mentioned above.

Alternatively, instead of incorporating these agents into polymeric particles, it is possible to entrap these materials in microcapsules prepared, for example, by coacervation techniques or by interfacial polymerization, for example, hydroxy-methylcellulose or gelatin-microcapsules and poly(methylmethacrylate) microcapsules, respectively, or in colloidal drug delivery systems, for example, liposomes, albumin microspheres, microemulsions, nanoparticles, and nanocapsules or in macroemulsions.

To be effective therapeutically as central nervous system targets, Leukocyte Interleukin, Injection (LI) or Multikine® should also readily penetrate the blood-brain barrier when peripherally administered. Compounds which cannot penetrate the blood-brain barrier can be effectively administered by an intraventricular route or other appropriate delivery system suitable for administration to the brain.

Leukocyte Interleukin, Injection (LI) or Multikine® may also be supplied in the form of a kit, alone, or in the form of a pharmaceutical composition as described above. Administration of LI can be conducted by conventional methods. For example, LI can be used in a suitable diluent such as saline or water, or complete or incomplete adjuvants. LI can be administered by any route appropriate for immune system stimulation, such as intravenous, intraperitoneal, intramuscular, subcutaneous, nasal, oral, rectal, vaginal, and the like.

As noted above, Leukocyte Interleukin, Injection (LI) or Multikine® may be for either a prophylactic or therapeutic purpose. When provided prophylactically, LI is provided in advance of any evidence or in advance of any symptom due to disease. When provided therapeutically, LI is provided at (or after) the onset of the disease or at the onset of any symptom of the disease. The therapeutic administration of LI serves to attenuate the disease and improves conventional treatment outcomes.

### Mode of action and characterization of Leukocyte Interleukin, Injection (LI) or Multikine®

Leukocyte Interleukin, Injection (LI) or Multikine® is a biologically active, minimally toxic, immunomodulatory mixture of naturally derived and naturally occurring human cytokines produced under set conditions as described herein. LI can be used as an anti-cancer and anti-viral therapy or as a neo-adjuvant therapy with a broad-spectrum application for cancer, infectious disease, and other diseases states responding to immunomodulation.

Animal studies demonstrate that "mixed interleukins" have immunomodulatory and immunostimulatory activity *in vitro* (Hadden et al., "Mixed Interleukins and Thymosin Fraction V Synergistically Induce T Lymphocyte Development in Hydrocortisone-Treated Aged Mice", Cell. Immunol. 144:228-236 (1992)). Without being limited to any one theory, it is hypothesized that the local/regional injection of "mixed interleukins" overcomes local immuno-suppression. Subsequently, a break of tolerance to tumor antigens occurs and allows for an effective local anti-tumor immune response to occur. It has also been known that the local instillation of interleukins in the region of the tumor or the actual transfection of Interleukin genes into a tumor markedly augments the anti-tumor immune response resulting in tumor regression as reported by Golumbek et al., "Treatment of Established Renal Cancer by Tumor Cells Engineered to Secrete Interleukin-4", Science 254:713-716 (1991).

One hypothesis is that the LI mode of action involves the combined activity of the different cytokines present in LI inducing a cascade of events. First, tumor necrosis factors present in LI such as TNF-α attack the tumor to release tumor antigens. Second, antigen presenting cells (*e*.*g*., dendritic cells) transport the newly released tumor antigens to lymph nodes. Third, lymphoproliferative cytokines present in LI administered peri-tumorally and peri-lymphatically *e.g.* IL-1, IL-2 induce a marked polyclonal expansion of tumor specific T-cells primarily in lymph nodes. Fourth, LI recruits CD4⁺ T-cells from local lymph nodes via chemotactic factors and reverts the balance of intratumoral CD4⁺/CD8⁺ cells in favor of CD4+ T-cells, which further up-regulates the antitumor immune response resulting in tumor cell necrosis. Fifth, LI recruits neutrophils from the circulation via GM-CSF, also present in LI, which propagate the destruction of the tumor cell nests. Sixth, either LI-derived cytokines or the *de novo* cytokine production by the tumor infiltrating cells induce massive local fibrosis.

Quite unexpectedly, the administration of Leukocyte Interleukin, Injection (LI) or Multikine® pre-surgery leads to an increase in the number of tumor cells in the cell cycle phase without increasing the risk of a more rapidly growing and more rapidly recurring tumor as would otherwise be predicted from the art. The ability to induce tumor cells into cell-cycle appears to be unique to LI and may be due to the synergistic effect of the different cytokines present in this investigational drug and the differential effect of these cytokines on both the host's immune system and the tumor cells (Timár et al., "The effect of Leukocyte Interleukin, Injection on the peri- and intratumoral subpopulation of mononuclear cells and on tumor epithelia - A possible new approach to augmenting sensitivity to radiation and chemotherapy in oral cancer. A multi-center Phase I/II clinical trial", The Laryngoscope (2003)).

Data regarding the recurrence rate of patients treated with Leukocyte Interleukin, Injection (LI) or Multikine® prior to surgery showed no recurrence of cancer at 24 months post treatment with LI. Remarkably, a small cohort of 8 sequentially treated patients did not have a single recurring patient in the 24 months follow-up period. In stark contrast, the literature teaches the recurrence rate of similar patients at about 50% at 18-24 months post surgery.

In particular, Leukocyte Interleukin, Injection (LI) or Multikine® treatment did not appear to induce active proliferation of tumor residing lymphoid cells. Correspondingly, stromal Ki-67⁺ cells decreased while the frequency of Ki-67⁺ cancer cells increased following LI treatment. Thus, LI treatment induced an increase in the number of cycling tumor cells leading to increased susceptibility of the residual tumor to follow-on treatment with radiation and/or chemotherapy. Although other studies conducted with both natural and recombinant cytokines have shown efficacy in the treatment of cancer therapy, those studies have failed to teach the induction of entry into the cell cycling phase or the new method of synergistically combining LI with chemotherapy, immuno-therapy and radiation therapy (Timár et al., "The effect of Leukocyte Interleukin, Injection on the peri- and intratumoral subpopulation of mononuclear cells and on tumor epithelia - A possible new approach to augmenting sensitivity to radiation and chemotherapy in oral cancer. A multi-center Phase I/II clinical trial", The Laryngoscope (2003)).

For example, studies with the use of natural human and recombinant IL-2 and other cytokines as well as in local-regional therapy demonstrated the immune augmenting and anti-cancer activity at various sites. In particular, IL-2 demonstrates activity in the pleural cavity, liver and the urinary bladder while IFN-α demonstrates activity in the ovary while IFN-β demonstrates activity in the brain (Yasumoto et al., "Induction of lymphokine-activated killer cells by intrapleural instillations of recombinant interleukin-2 in patients with, malignant pleurisy due to lung cancer", Cancer Res 47:2184-7 (1987); Mavilgit et al., "Splenic versus hepatic artery infusion of interleukin-2 in patients with liver metastases", J Clin Onco/ 8:319-24 (1990); Pizza et al., "Tumor regression after intralesional injection of interleukin-2 (IL-2) in bladder cancer. Preliminary report", Int J Cancer 34:359-67 (1984); Berek et al., "Intraperitoneal recombinant α-interferon for "salvage" immunotherapy in stage III epithelial ovarian cancer: a gynecologic oncology group study", Cancer Res 45:4447-53 (1985); and Fettel et al., "Intratumor administration of beta-interferon in recurrent malignant gliomas-A phase I clinical and laboratory study", Cancer 65:78-83 (1990)). Even further, IFN-γ has been shown to demonstrate activity in skin while TNF-α demonstrates activity in the genitalia while a mixture of various cytokines demonstrates activity in the head and neck (Edwards et al., "The effect of intralesional interferon gamma on basal cell carcinomas", J Am Acad Dermatol 22:496-500 (1990); Irie et al., "A case of vulva cancer responding to the recombinant human tumor necrosis factor (PT-950) local injection therapy", Gan No Rinsho 34:946-50 (1988); and Pulley et al., "Intravenous, intralesional and endolymphatic administration of lymphokines in human cancer", Lymph Res 5:S157-63 (1986)). Moreover, studies of recombinant IL-2 administrations for 10 days prior to surgery in the jugular peri-lymphatic or jugular peri-lymphatic and under the chin showed variable necrosis and lymphocytic infiltration (Valente et al., "Infiltration leukocyte polulations and T-lymphocyte subsets in head and neck squamous cell carcinomas from patients receiving perilymphatic injections of recombinant interleukin-2", Mod Pathol 3:702-8 (1990); DeStefani et al, "Treatment of oral cavity and oropharynx squamous cell carcinoma with perilymphatic interleukin-2: clinical and pathologic correlations", J Immunother 19:125-33 (1996)). Moreover, microscopic examination of the resected tumors demonstrated an increase in lymphocytic infiltrate correlating to the clinical observations of IL-2 (Saito et al., "Immunohistology of tumor tissue in local administration of recombinant interleukin-2 in head and neck cancer, Nip Jibi Gakkai Kaiho 92:1271-6 (1989).

Nevertheless, none of the aforementioned studies showed any change in the gross dimensions of resected tumors despite two remissions at putatively high doses of recombinant IL-2 (800,000 U for four weeks [U=Units} in 20 head and neck patients (Saito et, al., "Clinical evaluation of local administration of rIL-2 in head and neck cancer", Nip Jibi Gakkai Kaiho, 921271-6 (1989)). Furthermore, the aforementioned studies have been limited by the small number of patients and hampered by the lack of a pathological comparison to a control group.

Although a recent randomized multi-centre phase III study of 202 OSCC patients by De Stefani *et al.* indicated that peri-lymphatic administration of low doses (5000U/day [U=Units] of recombinant human IL-2 for 10 days prior to surgery, into the ipsilateral cervical lymph node chain, resulted in a significant (p<0.01) increase in disease-free survival, which in turn resulted in longer overall survival (p<0.03), De Stefani *et al.* failed to assess the role of this treatment regimen on cell cycling and its effect on the improvement of radiation and chemotherapy. *See* De Stefani et al., "Improved Survival With Perilymphatic Interleukin 2 in Patients With Resectable Squamous Cell Carcinoma of the Oral Cavity and Oropharynx", Cancer 95: 90-97 (2002). Furthermore, despite teaching 5000U/day, no comparisons between the present invention and De Stefani *et at.* could be made with regard to De Stefani *et al.*'s teaching of a "high" and "low" dose of administered biologic because the drug potency was measured by an underfinable U (Units). In contrast, the present inventors have validated and completed, the full USP analytical methods validation program for determining the biological
activity of Multikine® in IU (International Units).

### Drug Safety, Pilot Efficacy and Composition

Leukocyte Interleukin, Injection (LI) or Multikine® has been tested in over 200 Cancer, HIV, and HIV/HPV infected, patients with no severe adverse events related to LI administration, the preparation of which is described in U.S. Patent application 10/611,914 and the contents thereof being incorporated herein by reference (Harris et al., "Immunologic approaches to the treatment of prostate cancer", Semin Oncol, Aug;26(4):439-7 (1999); Timár et al., "The effect of Leukocyte Interleukin, Injection on the peri- and intratumoral subpopulation of mononuclear cells and on tumor epithelia - A possible new approach to augmenting sensitivity to radiation and chemotherapy in oral cancer. A multi-center Phase I/II clinical trial", The Laryngoscope 2003; Brown et al., "A Phase I Open-Label Study of Leukocyte Interleukin, Injection in HIV-1 infected individuals: preliminary evidence for improved delayed-type hypersensitivity responses to recall antigens", Antiviral Therapy 5 (supplement) 18,2000; Taylor et al., "Immunotherapy with Leukocyte Interleukin, Injection for human papilloma virus (HPV) induced cervical dysplasia in HIV patients", Annual Meeting of the International Society for Interferon and Cytokine Research, Cleveland, OH, October 2001; Taylor et al., "Immunotherapy with Leukocyte Interleukin, Injection for human papilloma virus (HPV) induced cervical dysplasia in HIV patients", 33rd SGO Conference, Miami, FL, March (2002)). Leukocyte Interleukin, Injection (LI) or Multikine® was also shown to be safe in animal toxicological studies in mice, rats, guinea pigs and dogs. Furthermore, LI was tested for and has demonstrated pilot efficacy in head and neck cancer and cervical dysplasia.

### Clinical Study in Patients Suffering from Head and Neck Cancer treated with Leukocyte Interleukin, Injection (LI) or Multikine®

### Patients

Forty-one patients were treated with Leukocyte Interleukin, Injection (LI) or Multikine® wherein twenty-one patients (mean age: 59.4 years, range: 40-87) with previously untreated head and neck cancer were included. The inclusion criteria were: >18 years; able and willing to provide written informed consent; histologically confirmed squamous cell carcinoma of the oral cavity (locations: floor of the mouth 7, tongue 8, lip 1, oropharynx 1, bucca 1 and gingiva 1); no known distant (visceral) metastatic disease; life expectancy of more than 6 months. Patients who were pregnant or had radiation to the site of LI administration, duodenal or gastric ulcer, or asthma were excluded.

Of the 21 LI treated patients 19 were evaluable. Two of the treated patients were enrolled and subsequently confirmed as having anaplastic carcinoma of the uvula and adenocarcinoma of the floor of the mouth, respectively. These two patients were given the full course of therapy including the administration of Leukocyte Interleukin, Injection (LI) but were not part of the histopathology/ immunohistochemistry analysis.

Historical controls were selected for the pathological examination portion of this study, exclusively from the pathology specimen repository of the National Institute of Oncology, Budapest, Hungary, and were matched to the specimens from the LI-treated group based on the size and location of the tumor, tumor stage, sex and age of the patients. The control group consisted of 20 patients (mean age: 57.5 years, range: 40-77), all with biopsy-confirmed oral squamous cell carcinoma of the oral cavity (locations: floor of mouth 3, tongue 12, lip 5).

### Patient examination

Prior to the inclusion in the trial patients underwent a general assessment. Their medical history was also reviewed. Once enrolled a complete physical examination, hematological, blood chemistry workup, chest cardioradiography and electrocardiogram were performed. To visualize and measure oral cavity tumor, both physical measurement and magnetic resonance imaging (MRI) were performed. Oral cancers surface area (PE measurement) was measured in the longest dimension and at right angle to the longest dimension at the largest diameter of the palpable tumor. For MRI, coronal, axial, and sagittal scans of T1-,T2-weighted fast spin echo (FSE), inversion recovery FSE (IRFSE) and fat saturated T1-weighted sequence with gadolinium were applied. Oral cancers were measured at the regions of highest diameter. MRI analysis of the OSCC was performed before and after LI treatment in each case, and evaluated with a standard method. In the responder group, LI treatment decreased tumor size as well as T2 and IRFSE signal intensity, the level of contrast enhancement and the margin of the lesions became less conspicuous. In the non-responder group MR morphology revealed no significant alteration in the tumor following LI treatment. Increasing tumor size was shown by pathology/morphometry for cases of progression. Patients were interviewed at each subsequent visit and asked about quality of life (*e.g.,* level of pain, degree of tongue mobility, *etc*.) using a qualified questionnaire. The treating physician assessed toxicity at each visit.

### Treatment protocol

Leukocyte Interleukin, Injection (LI) or Multikine® administration can be performed with any of the following doses of 200, 400, 800, 1200, 1600, 2400, 3200, 4800, 8000, 9600 and 12000 IU. The precise dose of vaccine preparation to be employed in the formulation depends on the route of administration, and the nature of the patient, and should be decided according to the judgment of the practitioner and each patient's circumstances according to standard clinical techniques. An effective amount is that amount sufficient to produce a favorable response in the host to which the vaccine preparation is administered.

### Pathology

Pathological evaluation was performed in a central pathology laboratory at the Department of Tumor Progression, National Institute of Oncology (Budapest, Hungary). A single pathology protocol governed this study, and described the fixation of the surgically excised specimens and the gross, macroscopic and microscopic examination, histology and immunohistochemistry procedures as provided in Tímár et al., "The effect of Leukocyte Interleukin, Injection (Multikine) treatment on the peritumoral and intratumoral subpopulation of mononuclear cells and on tumor epithelia: a possible new approach to augmenting sensitivity to radiation therapy and chemotherapy in oral cancer - a multicenter phase I/II clinical trial", Laryngoscope 113:2206-2217 (2003).

Diagnosis of the oral lesions was based on an excision biopsy of the suspected lesion, and cancers classified as T2-3N0-2M0 were selected for immunotherapy with the LI treatment regimen (Hadden et al., "Interleukins and contrasuppression induce immune regression of head and neck cancer", Arch Otolaryngol Head Neck Surg 120:395-403 (1994)). At the end of the LI treatment period, and prior to surgery, clinical responses were evaluated as described above and patients were scheduled for tumor resection. The excised tissue was placed in pre-labeled containers with buffered formalin, fixed overnight prior to embedding the tissue in paraffin and preparing thin section slides for H&E staining and immunohistochemistry.

Histology and AJCC grading was performed from H&E stained sections. The histopathological analysis was done on 3 different tumor regions: surface (R1), center (R2), and tumor-stroma interface (R3). Occurrence of necrotic tumor cells was also evaluated using H&E slides. The percent of the epithelial component versus the stroma in the OSCC tumors was determined by two methods; connective tissue was stained according to Mallory (trichrome staining) and the slides were measured for the area of cancer nests (tumor epithelia) by using ImagePro analysis software (Media Cybernetics, Silver Spring, MD). Separately, slides containing resected tissue were labeled for cytokeratine immunohistochemically using pan-cytokeratine antibody (DAKO, Glostrup, Denmark; A1A3+CK19), which labels cancer cells.

### Characterization of the mononuclear cell infiltrate

Mononuclear cells present in the close vicinity of tumor cell nests were determined by using immunohistochemistry performed on paraffin-embedded sections of the tumor samples after de-paraffination and microwave antigen retrieval. Antibodies against the following markers were applied: myeloperoxidase, HLA class II (HLA-DP, DQ, DR), CD8, CD20, CD34, CD45R0, CD68 (all from DAKO); CD4, CD25, CD56 (Novocastra Laboratories Ltd., Newcastle upon Tyne, UK); CD1a (Immunotech, Marseille, France); and CD134 (PharMingen, San Diego, CA). In all cases negative control slides were prepared using isotype-matched non-immune Igs.

Immunohistochemical (IHC) labeling was performed with the DAKO LSAB-2 kit using a biotinylated anti-mouse/anti-rabbit IgG linker and Streptavidin-HRP to reveal specifically bound antibodies. The chromogen used was AEC (red) label. Sections were counterstained for the nuclei with hematoxylin.

### Evaluation of tumor samples

Tumor samples from the Leukocyte Interleukin, Injection (LI) treated group and control (non-LI-treated) group were evaluated for histological changes and for the infiltrating cells: T and B lymphocytes, NK cells, dendritic cells, macrophages and neutrophils, hematopoietic stem cells, as well as T-cell activation and memory cell markers. A 40x magnification was used to select the area and a 400x magnification for the identification and quantitation of infiltrating cells. The density of mononuclear cells was determined based on the "hot spot" technique, which means that in each studied tumor area density was measured at the region of the highest tumor-infiltrating mononuclear cell density. This method minimizes the problem of the extreme heterogeneity of the cellular infiltrates in tissues.

The histologic evaluation of each patient's tumor section was performed by 3 independent pathologists and, in case of disagreement, consensual determinations were reached. Morphometric measurements were performed by the same 3 pathologists and done without the knowledge of the clinical background and treatment outcome of each of the cases.

### Statistical analysis

Pathology data was analyzed by ANOVA single factor analysis and χ² test, and a 'p' value less than 0.05 was considered to be statistically significant.

The following are non-limiting examples of the various dosages capable of being used in the present invention wherein the Example 1 relates to the studied dosage. However, it will be understood that the invention will not be limited in any way to just the dosages provided and that other effective dosages may be used to treat patients in need thereof.

### Example 1

One half of a daily dose of 800 IU as IL-2 was injected peritumorally. The other half (400 IU) was injected perilymphatically sequentially and at the same visit. Both injections are administered over a three-week period, 5 times per week, reaching a cumulative dose of 12,000 IU, as IL-2. LI is administered intradermally at the circumferential margins of the visible/palpable tumor mass. The perilymphatic injections are given at the posterior mandibular area in the area of the jugular lymphatic chain ipsilateral to the injected tumor mass.

A single intravenous (bolus) infusion of cyclophosphamide is given, Inj., 300 mg/m², three days prior to the first LI administration. Indomethacin (25 mg) is self-administered orally (with food), three times daily for a total daily dose of 75 mg beginning 3 days post cyclophosphamide administration and until 24 hours prior to surgery. Zinc Sulfate (50 mg, as elemental Zinc) and multivitamin supplement, once daily, is self-administered beginning 3 days after cyclophosphamide administration and until 24 hours prior to surgery.

### Cyclophosphamide

Cyclophosphamide, Inj. USP (Bristol-Myers-Squibb, UK) is supplied as a sterile powder containing 45 mg sodium chloride, 75 mg mannitol or approximately 82 mg sodium bicarbonate per 100 mg cyclophosphamide for reconstitution prior to intravenous infusion.

### Indomethacin

Indomethacin USP (Sanofi - Synthelabo, France) is supplied as 25 mg tablets for oral self-administration with food.

### Zinc Sulfate and Multivitamins

Zinc sulfate (50 mg as elemental Zinc, R. P. Scherer Corporation, Clearwater, Florida, USA) and OTC multivitamins are supplied by the clinic to each patient for self-administration.

### Example 2

One half of a daily dose of 1200 IU as IL-2 can be injected peritumorally. The other half (600 IU) is injected perilymphatically sequentially and at the same visit. Both injections are administered over a three-week period, 5 times per week or less dependant on the total desired cumulative dose. LI is administered intradermally at the circumferential margins of the visible/palpable tumor mass. The perilymphatic injections are given at the posterior mandibular area in the area of the jugular lymphatic chain ipsilateral to the injected tumor mass. The remaining treatment protocol is the same as in Example 1.

### Example 3

One half of a daily dose of 1600 IU as IL-2 can be injected peritumorally. The other half (800 IU) is injected perilymphatically sequentially and at the same visit. Both injections are administered over a three-week period, 5 times per week or less dependant on the total desired cumulative dose. LI is administered intradermally at the circumferential margins of the visible/palpable tumor mass. The perilymphatic injections are given at the posterior mandibular area in the area of the jugular lymphatic chain ipsilateral to the injected tumor mass. The remaining treatment protocol is the same as in Example 1.

### Example 4

One half of a daily dose of 2400 IU as IL-2 can be injected peritumorally. The other half (1200 IU) is injected perilymphatically sequentially and at the same visit. Both injections are administered over a three-week period, 5 times per week or less dependant on the total desired cumulative dose. LI is administered intradermally at the circumferential margins of the visible/palpable tumor mass. The perilymphatic injections are given at the posterior mandibular area in the area of the jugular lymphatic chain ipsilateral to the injected tumor mass. The remaining treatment protocol is the same as in Example 1.

### Example 5

One half of a daily dose of 3200 IU as IL-2 can be injected peritumorally. The other half (1600 IU) is injected perilymphatically sequentially and at the same visit. Both injections are administered over a three-week period, 5 times per week or less dependant on the total desired cumulative dose. LI is administered intradermally at the circumferential margins of the visible/palpable tumor mass. The perilymphatic injections are given at the posterior mandibular area in the area of the jugular lymphatic chain ipsilateral to the injected tumor mass. The remaining treatment protocol is the same as in Example 1.

### Example 6

One half of a daily dose of 4800 IU as IL-2 can be injected peritumorally. The other half (2400 IU) is injected perilymphatically sequentially and at the same visit. Both injections are administered over a three-week period, 5 times per week or less dependant on the total desired cumulative dose. LI is administered intradermally at the circumferential margins of the visible/palpable tumor mass. The perilymphatic injections are given at the posterior mandibular area in the area of the jugular lymphatic chain ipsilateral to the injected tumor mass. The remaining treatment protocol is the same as in Example 1.

### Example 7

One half of a daily dose of 9600 IU as IL-2 can be injected peritumorally. The other half (4800 IU) is injected perilymphatically sequentially and at the same visit. Both injections are administered over a three-week period, 5 times per week or less dependant on the total desired cumulative dose. LI is administered intradermally at the circumferential margins of the visible/palpable tumor mass. The perilymphatic injections are given at the posterior mandibular area in the area of the jugular lymphatic chain ipsilateral to the injected tumor mass. The remaining treatment protocol is the same as in Example 1.

### Example 8

One half of a daily dose of 12000 IU as IL-2 can be injected peritumorally. The other half (6000 IU) is injected perilymphatically sequentially and at the same visit. Both injections are administered over a three-week period, 5 times per week or less dependant on the total desired cumulative dose. LI is administered intradermally at the circumferential margins of the visible/palpable tumor mass. The perilymphatic injections are given at the posterior mandibular area in the area of the jugular lymphatic chain ipsilateral to the injected tumor mass. The remaining treatment protocol is the same as in Example 1.

### Clinicopathological response

No systemic or local toxicity or severe adverse events related to Leukocyte Interleukin, Injection (LI) are present for a daily dose of 800 IU/mL injections administered over a three-week period, 5 times per week for a cumulative dose of 12,000 IU/mL being the Example 1. No complications at surgery, post-surgery, or wound healing related to LI were reported. Primary tumor samples of 19 (LI) treated and 20 control (non-LI-treated) histologically matched squamous cell carcinoma patients were evaluated. In the case of the LI-treated group, surgical resection of the primary tumor was performed between days 14 and 54 after the end of the LI treatment - median time to surgery, 22 days. The histological diagnosis, keratinization (Brodes scores) and TNM stage of all the LI-treated and control patients are listed in Table 1 shown below. The two groups did not differ histologically nor with respect to keratinization or TNM stages.

**Table 1**

| Pathology data of the OSCC patients | | | | |
|---|---|---|---|---|
| **Control group** | **Diagnosis** | **Grade** | **pTNM** | **Necrosis** |
| 1 | cc.spinocell. | BR3 | PT2N0M0 | - |
| 2 | CPC | BR2 | PT2N0M0 | macroscopic |
| 3 | CPC | BR3 | PT2N1M0 | - |
| 4 | CPC | BR3 | PT2N0M0 | unicellular |
| 5 | CPC | BR2 | PT2N0M0 | - |
| 6 | CPC | BR3 | PT2N2M0 | macroscopic |
| 7 | CPC | BR2 | PT2N0M0 | - |
| 8 | CPC | BR3 | PT3N0M0 | macroscopic |
| 9 | CPC | BR3 | PT2N0M0 | - |
| 10 | CPC | BR3 | PT2N0M0 | - |
| 11 | CPC | BR1 | PT2N0M0 | microscopic |
| 12 | CPC | BR1 | PT2N0M0 | - |
| 13 | CPC | BR1 | PT3N0M0 | - |
| 14 | CPC | BR3 | PT2N0M0 | - |
| 15 16 | CPC | BR1 | PT2N0M0 | macroscopic |
| | CPC | BR3 | PT3N0M0 | - |
| 17 | CPC | BR1 | PT2N0M0 | microscopic |
| 18 | CPC | BR2 | PT2N0M0 | - |
| 19 | CPC | BR1 | PT2N0M0 | - |
| 20 | CPC | BR1 | PT2N0M0 | unicellular |

| **LI-Treated group** | | | | |
|---|---|---|---|---|
| 1 | CPC | BR3 | T2N0M0 | microscopic,MF |
| 2 | CPC | BR1 | T2N0M0 | - |
| 3 | CPC basaloid | BR3 | T2N0M0 | microscopic,MF |
| 4 | CPC | BR3 | T2N0M0 | microscopic,MF |
| 5* | **fibrous stroma** | | | - |
| 6 | CPC | BR3 | T3N0M0 | - |
| 7 | CPC | BR2 | T1N0M0 | microscopic,MF |
| 8 | CPC | BR2 | T2N0M0 | - |
| 9 | CPC | BR2 | T2N0M0 | - |
| 10 | CPC | BR2 | T2N0M0 | microscopic,MF |
| 11 | CPC | BR2 | T2N0M0 | microscopic,MF |
| 12 | CPC | BR3 | T2N2M0 | microscopic,MF |
| 13 | CPC | BR3 | T2N1M0 | - |
| 14* | dysplasia | | | - |
| 15 | CPC | BR3 | T2N0M0 | - |
| 16 | CPC | BR2 | T2N0M0 | microscopic,MF |
| 17 | CPC | BR1 | T2N0M0 | **microscopic,MF** |
| 18 | CPC | BR1 | T2N0M0 | - |
| 19 | CPC | BR1 | T2N0M0 | microscopic,MF |

| | | | | |
|---|---|---|---|---|
| *= pathologically no tumor tissue detected. CPC= carcinoma planocellulare, BR= Brodes grading, MF= multifocal, OSCC= oral squamous cell carcinoma, pTNM= Pathology confirmed TNM classification | | | | |

For two patients within the LI-treated group indicated as no.'s 5 and 14 in Table 1, no cancer tissue was detected in the surgically resected "tumor" mass. These patients were considered to be complete responders exhibiting 100% tumor reduction by histopathology as a result of LI treatment regimen. In 2 other LI treated patients shown as no.'s 7 and 18 in Table 1, the use of an imaging technique verified >50% tumor volume reduction considered as partial (major) response. For 4 other patients indicated as no.'s 4, 6, 9 and 12 in Table 1, the volume reduction was proven to be >30%, which is considered as a minor response. Progressive disease with ≥40% tumor volume increase occurred in only one case (No. 17) while stable disease was detected in the rest of the cases (10/19). Therefore, the response rate in the LI-treated group was 21% with an overall response of 42% (8/19). The full pathological analysis of the effect of LI treatment on OSCC was performed on the LI-treated non-responder subgroup (stable disease and progressive disease, n=11) as well as separately on the LI-treated responder subgroup (n=6) excluding the 2 complete responders where no tumor tissue could be detected.

### Tumor-infiltrating lymphocytes

CD20⁺ B-cells were only present in the stroma of OSCC for a daily dose of 800 IU/mL injections administered over a three-week period, 5 times per week for a cumulative dose of 12,000 IU/mL being the Example 1. In tumor samples obtained following LI pre-treatment, a non-significant decrease in B-cells in OSCC irrespective of the tumor area was present. Clinical responses did not influence this pattern of B-cells in the OSCC.

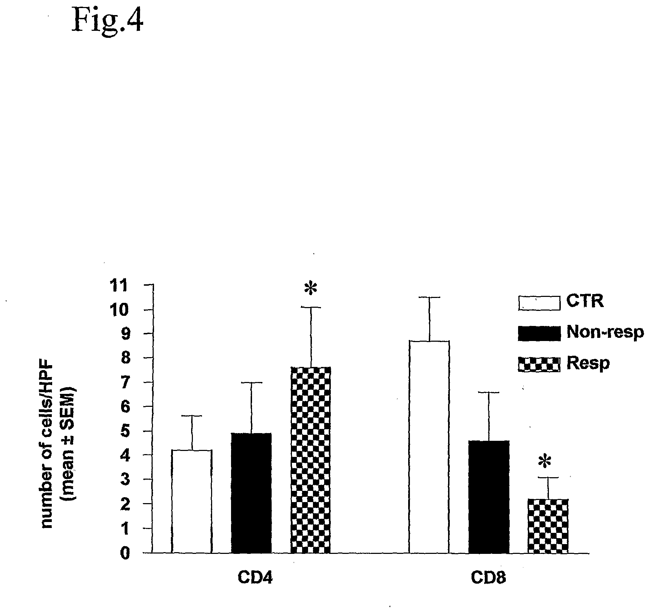

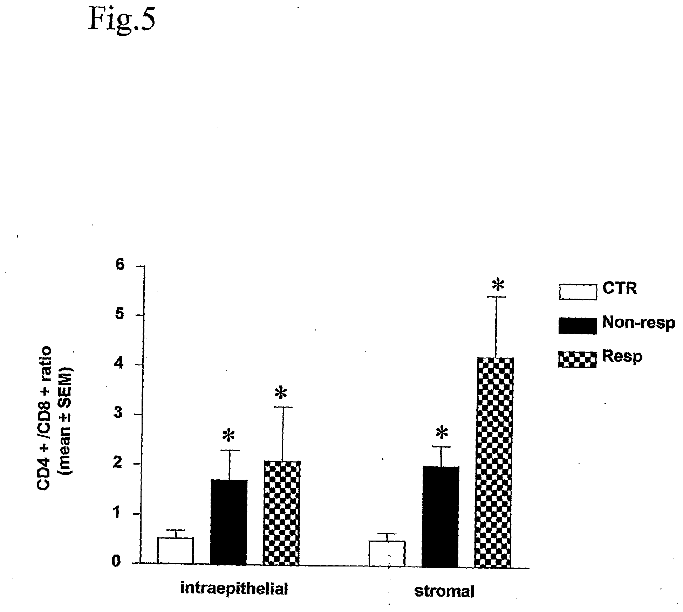

In the control group, CD8⁺ cells infiltrating the tumor and stroma outnumbered the CD4⁺ cells as shown in Fig.'s 1-4. As a result, the CD4⁺/CD8⁺ ratio was well below 1 at about 0.5 as shown specifically in Fig. 5. In the LI treated group, an increase in the density of CD4⁺ T-cells and decrease in the density of CD8⁺ T-cells was observed in the tumor stroma as shown in Fig. 4. A similar trend was seen in the case of intraepithelial CD4⁺ and CD8⁺ cells shown in Fig. 4. It is of note that these changes were significant only in the case of responders to LI treatment (p<0.05). As a consequence, the CD4⁺/CD8⁺ ratio was 2-8-fold higher in the cellular infiltrate of the LI-treated tumors as compared to the control group as shown in Fig. 5. Although the highest CD4+ and the lowest CD8+ cellular infiltrate densities were detected in the responder to LI treatment subgroup, the difference from non-responders (to LI-treatment) was only statistically significant (p<0.05) in the tumor stroma segment of the tumor, as can be seen by the CD4+/CD8+ ratio.

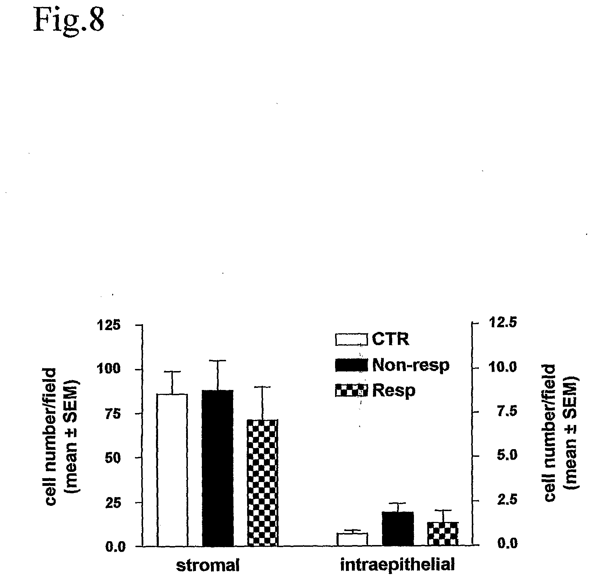

Evaluation of the number of activated T-cells based on the expression of CD25 and OX40 (CD134) molecules revealed a relatively low mean density of cells expressing these markers in the infiltrates of OSCC especially in the case of OX40 and no significant differences in their frequency between the control and LI-treated groups as shown in Fig.'s 6 and 7. However, using available frozen samples and immunofluorescence, CD25⁺/CD4⁺ T cells were more frequent in the LI-treated group for 10 out of 10 patients ranging 14-61% of CD4⁺ cells but were only detected occasionally in the control group for 1 out of 9 patients. The density of CD45R0⁺ memory cells is highly similar in the control and LI-treated cases in both the tumor stroma and intraepithelia as shown in Fig. 8. The density of CD45R0⁺ lymphoid cells was at an average ∼60% of T-cells (CD4⁺ + CD8⁺) suggesting that a high proportion of tumor infiltrating T lymphocytes were memory cells.

CD34⁺ hematopoietic stem cells and CD56⁺ NK cells were not found in the stroma or tumor epithelial nests of the OSCC, irrespective of the tumor area or the patient population investigated (control or LI-treated).

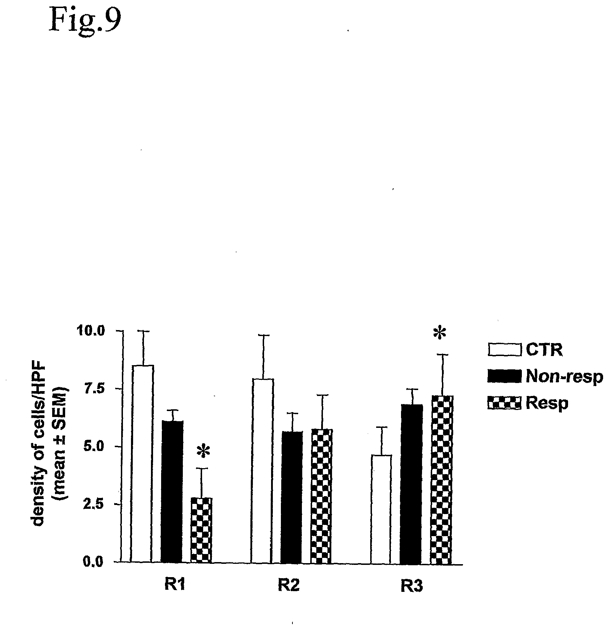

CD1a⁺ dendritic cells were almost exclusively located to the tumor epithelial nests. In the LI-responder subgroup, a differential effect of LI treatment on the distribution of dendritic cells in the 3 areas of OSCC was shown wherein no change was observed in the center of the tumor (R2) while a decrease (p<0.05) in CD1a⁺ dendritic cells was detected at the surface (R1), and an increase (p<0.05) in CD1a+ dendritic cells was present at the tumor-stroma interface (R3), as compared to control (non-LI-treated) samples as shown in Fig. 9.

### Inflammatory cells

Neutrophils were present both in the tumor stroma and in the tumor epithelial nest similar to macrophage distribution in the tumor while eosinophils were exclusively found in the tumor stroma for a daily dose of 800 IU/mL injections administered over a three-week period, 5 times per week for a cumulative dose of 12,000 IU/mL being the Example 1. The presence of neutrophil and macrophage infiltrate in the resected tumor specimens was determined by staining for myeloperoxidase (MPX) and CD68 markers, respectively.

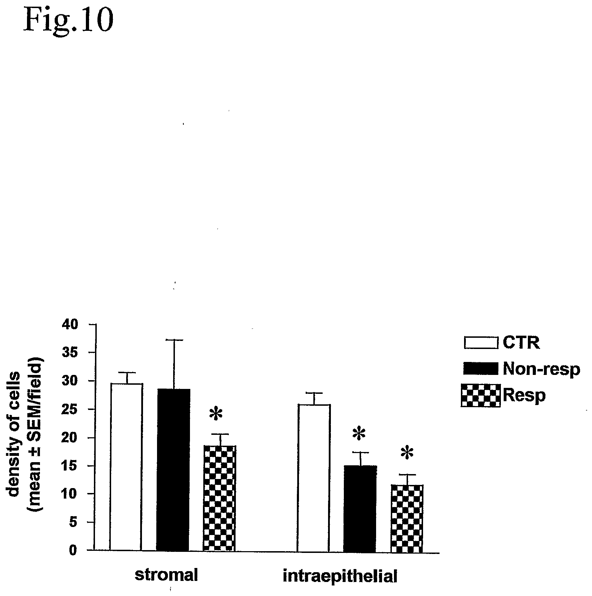

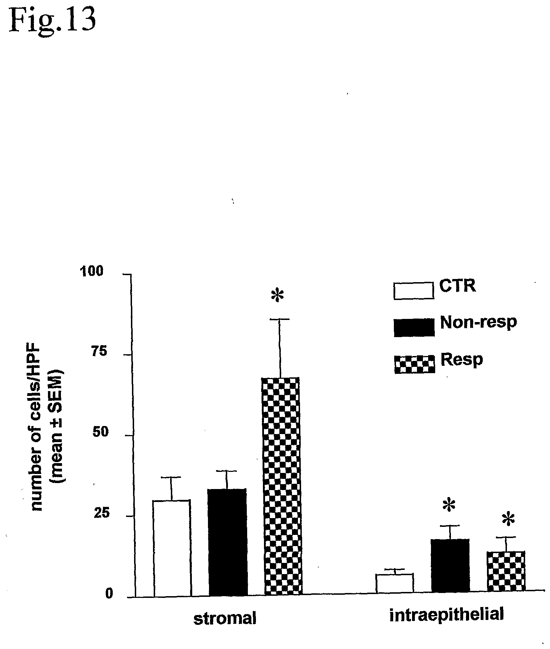

Analysis of the macrophage density indicated a down-modulation of the stromal presence of CD68⁺ macrophages exclusively in the LI responder group. This trend was even more pronounced intraepithelially in the LI-treated group (p<0.002 for the LI responder subgroup, and p<0.01 for LI non-responders) (Fig. 10). On the other hand, LI treated cases exhibited increased neutrophil migration into the cancer cell nests as shown in Fig.'s 11-13. Neutrophil density was also pronounced in the tumor stroma in the LI-responder subgroup as shown in Fig. 13.

In the LI-treated group, increased intratumoral infiltration by neutrophils was observed exclusively in cases with multifocal microscopic necrosis.

The density of eosinophils was found to be highly heterogeneous in both OSCC groups (LI-treated and control). Morphometric analysis revealed no significant alterations in eosinophilic cell population in the tumor stroma in the LI treated patients. However, a more detailed analysis of the cases revealed that tumors with high eosinophil density (>50 cells/HPF) were twice as numerous in the LI treated group for 9 out of the 19 patients or 47% compared to the controls of 5 out of 20 or 25%.

### Tumor necrosis

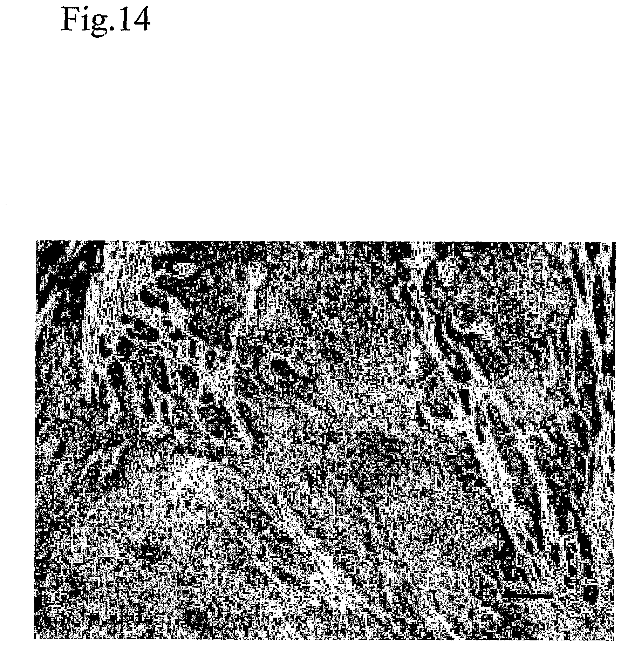

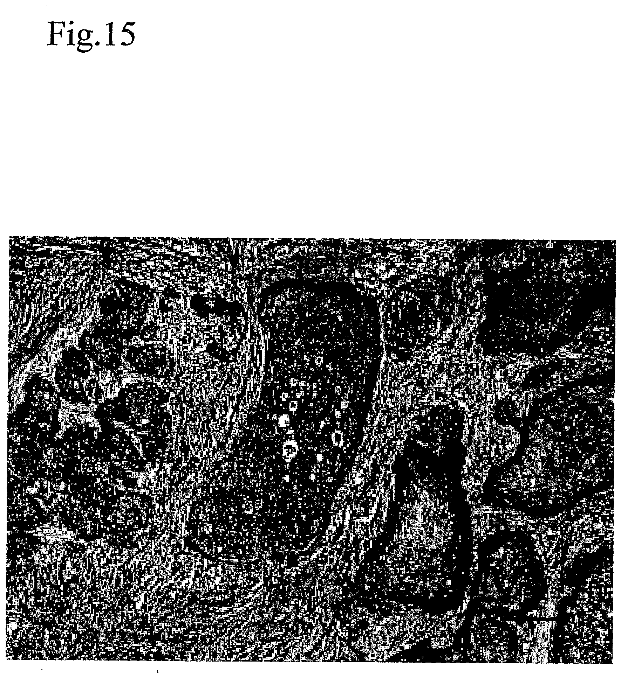

Macroscopic cancer necrosis foci in the surgically obtained samples occurred exclusively in the control group. However, multifocal microscopic necrosis was markedly more frequent in the LI-treated group for 10 of 17 patients as shown in the Table 1 and a Table 2 (shown below) and in a Fig. 15 compared to the control group for 4 out of 20 patients as shown in the Tables 1 and 2 and in a Fig. 14 (p<0.05). No significant difference in this respect between the responder and non-responder (to LI treatment) subgroups was seen.

**Table 2**

| Incidence of necrosis in OSCC cases | | | |
|---|---|---|---|
| | | LI-Treated | |
| Necrosis | CTR | Non-responders | Responders |
| None | 12/20 | 4/11 | 3/6 |
| Multifocal/microscopic | 4/20 | 7/11 | 3/6 |
| Macroscopic | 4/20 | 0/11 | 0/6 |

| | | | |
|---|---|---|---|
| Two complete remission cases have not been evaluated from the treated group. CTR= control. OSCC= oral squamous cell carcinoma. Non-responders vs. CTR: p<0.05, Non-responders + Responders vs. CTR: p<0.05 (χ² test) | | | |

### Tumor stroma / cancer cell nest ratio

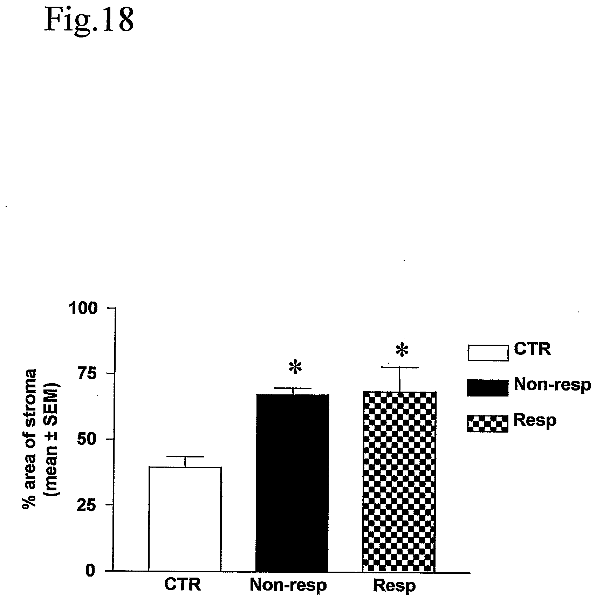

Leukocyte Interleukin, Injection (LI) treatment had a significant effect on the percent area of cancer cell nests to tumor stroma. The resected tissue samples were stained with Mallory trichrome technique where cancer cell nests were labeled in pink. The proportion of the stroma in the tumor tissue was determined by morphometry as shown in Fig.'s 16 and 17. Data indicated that the proportion of the collagenous stroma in tumor tissue in the LI-treated group was significantly increased as shown by Fig. 18 as compared to control samples (p<0.05) indicating a reduction of cancer cell nest area.

Analysis of the patterns of fibrosis in the OSCC samples showed that collagen fibers accumulated around the cancer cell nests and appeared as periepithelial staining. Collagen fibers were also found in between the cancer cell nests in the tumor stroma (Fig.'s 16 and 17). Periepithelial collagenosis was similar in frequency in both the control and LI-treated tumors while interstitial fibrosis was highly significantly (p<0.001) and more frequent in the LI-treated group for 12 out of 19 patients or 63% compared to the controls (2/20 or 10%). The non-responder and responder (to LI treatment) subgroups did not differ significantly in this respect as shown in Table 3.

**Table 3**

| Incidence and type of fibrosis in OSCC tumor samples | | | |
|---|---|---|---|
| | | LI-Treated | |
| Fibrosis | CTR | Non-responders | Responders |
| None | 8/20 | 0/11 | 0/8 |
| Periepithelial | 10/20 | 3/11 | 4/8 |
| Stromal | 2/20 | 8/11 | 4/8 |

| | | | |
|---|---|---|---|
| CTR= control, OSCC= oral squamous cell carcinoma. Non-responders vs. CTR: p<0.001, Responders vs. CTR: p<0.05, Non-responders + Responders vs. CTR: p<0.001 (χ² test) | | | |

### HLA class II expression

HLA class II expression of OSCC was determined by immunohistochemistry. Tumor-infiltrating cells exhibited very strong HLA II surface staining in both the LI-treated and control cases. The cancer cell expression was heterogeneous. In the control group, HLA II negative samples comprised about 11 of the 20 the cases while the remaining 9 cases contained tumors with at least a focal or a strong membrane HLA II expression as shown in Table 4. A similar pattern was observed in the LI-treated cohort (8/17 HLA II positive). However, in the LI-treated group, HLA II positive cases belonged exclusively to the non-responder group, while all LI-responder cases cancer cells were negative for HLA Class II antigen expression (p<0.05) (Table 4).

**Table 4**

| Expression of HLA class II in OSCC cancer cells | | | |
|---|---|---|---|
| | | LI-Treated | |
| Expression pattern | CTR | Non-responders | Responders |
| Negative | 11/20 | 3/11 | 6/6 |
| Focal | 6/20 | 4/11 | 0/6 |
| Strong/diffuse | 3/20 | 4/11 | 0/6 |

| | | | |
|---|---|---|---|
| Expression was determined by immunohistochemistry. Two complete remission cases have not been evaluated from the treated group. CTR= control, OSCC= oral squamous cell carcinoma. Non-responders vs. Responders: p<0.05 (χ² test) | | | |

### Discussion

Leukocyte Interleukin, Injection (LI) administered to advanced primary OSCC patients as a first-line treatment prior to conventional therapy such as surgery followed by radiation therapy induced alterations in infiltrating immune cells rather than in inflammatory cells. Changes in the T-cell population that migrated into the cancer cell nests and the infiltrating T-cells were found to be activated at the lowest LI dose administered demonstrated by increased CD25 expression. However, a treatment regimen of a low-dose LI, 400 IU as IL-2, 3 days/week for 2 weeks did not result in massive necrosis of the tumor tissue as shown by the lack of any marked change in the ratio of connective tissue to the tumor epithelial component.

However, for a higher daily dose of 800 IU/day as IL-2 for 5 days per week over 3 weeks that was administered to OSCC patients as first-line treatment prior to the initiation of conventional therapy, the LI-treated patients demonstrated a significant increase (p<0.05) in tumor infiltrating CD4+ T-cells with a concomitant decrease in CD8⁺ T-cells (*in situ*). This change resulted in a marked difference in the CD4⁺/CD8⁺ ratio for OSCC while the controls were characterized by a low CD4⁺/CD8⁺ ratio (<1, with CD8⁺ T-cell predominance).

A significantly higher CD4+/CD8+ ratio was observed in both the LI-treated non-responder and responder to LI treatment subgroups with a 2-8 fold increase with a CD4⁺ T-cell predominance. Notably, the stromal CD4+/CD8+ ratio was significantly higher (p<0.05) in the LI-responders as compared to non-responders suggesting clinical importance to this phenomenon.

A proportion of these CD4⁺ T-cells carried the activation marker CD25. The expression of OX40, a marker of freshly activated CD4⁺ T-cells which has been detected on a portion of TIL in several cancers including head and neck carcinomas was rare in the T-cell infiltrate (Vetto et al., "Presence of the T-cell activation marker OX-40 on tumor infiltrating lymphocytes and draining lymph node cells from patients with melanoma and head and neck cancers", Am J Surg 174:258-265 (1997); Ladányi et al., "T-cell activation marker expression on tumor-infiltrating lymphocytes as prognostic factor in cutaneous malignant melanoma", Clin Cancer Res 10:521-530 (2004)). The lack of Ox40 expression suggest a later phase of the T-cell activation process. This may be due to the relatively long period in the range of 14-54 days with a median of 22 days between the end of LI therapy and the surgical removal of the tumors in the LI-treated group.

On the other hand, double-labeling studies indicated that a large proportion of the CD4⁺ and CD8⁺ T-cells were CD45R0⁺ suggesting that they were memory T-cells. Parallel to these changes, intraepithelial dendritic cells shifted from the surface of the OSCC to the tumor-stroma interface. In a previous report on the local effect of IL-2 infusion in head and neck cancer, a similar selective effect on CD4⁺ T lymphocytes and dendritic cells was observed with a marked increase in CD25⁺ cells shown only at lower doses of IL-2 administration (Salter et al., "The phenotypic changes in tumour infiltrating lymphocytes and tumour cells following intra-arterial infusion of interleukin-2 in patients with squamous cell carcinoma", J Pathol 176:167-173 (1995)).

Leukocyte Interleukin, Injection (LI) treated OSCC patients were characterized by a markedly altered pattern of inflammatory cells suggesting that an acute inflammatory reaction was mediated predominantly by neutrophils and to a smaller extent eosinophils rather than by macrophages. Other studies in melanoma patients showed that the accumulation of neutrophil leukocytes at vaccination and tumor sites was prevalent after vaccination with GM-CSF producing autologous tumor cells (Soiffer et al., "Vaccination with irradiated autologous melanoma cells engineered to secrete human granulocyte-macrophage colony-stimulating factor generates potent antitumor immunity in patients with metastatic melanoma", Proc Natl Acad Sci USA 95:13141-13146 (1998); Soiffer et al., "Vaccination with irradiated, autologous melanoma cells engineered to secrete granulocyte-macrophage colony-stimulating factor by adenoviral-mediated gene transfer augments antitumor immunity in patients with metastatic melanoma", J Clin Oncol 21:3343-3350 (2003)). One explanation for increased neutrophil infiltration in the LI-treated patients as compared to controls is that LI preparations contain several cytokines including GM-CSF.

In parallel to the complex alterations in the tumor-infiltrating cellular components of the host antitumor response, multifocal necrosis of cancer cell nests and an increase in the proportion of connective tissue were detected in the LI-treated OSCC patients as compared to the non-treated controls. These changes may reflect the aftermath of an antitumor immune response raised against the OSCC induced by LI neoadjuvant administration.

A successful immune response to OSCC may require the reversal of the inherently low intratumoral CD4⁺/CD8⁺ ratio. The predominance of CD8⁺ over CD4⁺ cells in solid tumors is not specific for OSCC. Similar low CD4⁺/CD8⁺ ratios have been reported in basal cell carcinoma, cervical and breast cancers (Rohrbach et al., "Immunology and growth characteristics of ocular basal cell carcinoma", Graefes Arch Clin Exp Ophthalmol 239:35-40 (2001); Santin et al., "Tumor-infiltrating lymphocytes contain higher numbers of type 1 cytokine expressors and DR+ T cells compared with lymphocytes from tumor draining lymph nodes and peripheral blood in patients with cancer of the uterine cervix", Gynecol Oncol 81:424-432 (2001); Murta et al., "Lymphocyte subpopulations in patients with advanced breast cancer submitted to neoadjuvant chemotherapy", Tumori 86:403-407 (2000)). High percentages of CD4⁺ T cells or high CD4⁺/CD8⁺ ratios correlated with better prognosis or response to immunotherapy in melanoma, B-cell non-Hodgkin's lymphoma, renal cell carcinoma, and cervical carcinoma (Hakansson et al., "Tumour-infiltrating lymphocytes in malignant melanoma and response to interferon alpha treatment", Br J Cancer 74:670-676 (1996); Ansell et al., "CD4+ T-cell immune response to large B-cell non-Hodgkin's lymphoma predicts patient outcome", J Clin Oncol 19:720-726 (2001); Igarashi et al., "Effect of tumor-infiltrating lymphocyte subsets on prognosis and susceptibility to interferon therapy in patients with renal cell carcinoma", Urol Int 69:51-56 (2002); Sheu et al., "Reversed CD4/CD8 ratios of tumor-infiltrating lymphocytes are correlated with the progression of human cervical carcinoma", Cancer 86:15371543 (1999)).

This suggests that an acquired immunosuppressed state of cancer patients, induced by the tumor is a common phenomenon reflecting the general poor immune response in cancer patients.

Furthermore, CD4⁺ T lymphocytes play a central role in initiating and maintaining antitumor immunity by providing help for CD8⁺ CTL, stimulating antigen presenting cells and sustaining immunological memory or in some cases by performing effector functions via cytokine secretion or direct cytolysis (Goedegebuure et al., "The role of CD4+ tumor-infiltrating lymphocytes in human solid tumors", Immunol Res 14:119-131 (1995); Hung et al., "The central role of CD4+ T cells in the antitumor immune response", J Exp Med 188:2357-2368 (1998); Gao et al., "Antigen-specific CD4+ T-cell help is required to activate a memory CD8+ T cell to a fully functional tumor killer cell", Cancer Res 62:6438-6441 (2002)). In the case of oral carcinomas treated with Leukocyte Interleukin, Injection (LI), it is not clear which are the specific mediators (in LI) that elicit the anti-tumor immune response. However, the increased density of intraepithelial leukocytes in parallel to microscopic necrosis after LI treatment suggests that the activation of non-specific antitumor mechanisms (in OSCC patients) is equally important in order to produce clinico-pathologically detectable destruction of the established tumor.

Histopathological comparison of tumors in the LI-treated non-responder subgroup to those of the responder subgroup where clinical and histopathological regressions were observed in 8 of 19 cases revealed that most of the studied immunological parameters from immune cell composition to inflammatory cell patterns were similar. These data suggest that the LI-treated group responded homogenously to the treatment but that the anticancer effects of LI treatment were heterogeneous. A plausible explanation for this discrepancy could be individual differences in immunosensitivity of OSCC tumors such as differences in tumor antigens and/or HLA composition and/or the level of impairment of functional immune response in the different patients.

Analysis of HLA class II expression by OSCC cancer cells indicated that the level of expression was highly similar between the LI-treated and control group (∼45% positive for HLA Class II antigen expression). This suggests that LI treatment did not induce or enhance the expression of HLA II molecules on the tumor as compared to controls. However, within the LI-treated cohort, all HLA II positive cases belonged exclusively to the non-responder subgroup while all the LI responder cases were negative. Although derived from a small number of patients, this finding may indicate that HLA class II expression on OSCC cells may be associated with an unfavorable clinical outcome of disease or with a diminished ability to respond to immunotherapy. Similar findings were reported in malignant melanoma where the expression of MHC class II constitutes a poor prognostic factor and which may be due to the induction of T cell anergy by MHC class II expressing tumor cells in the absence of costimulatory signals (Brocker et al., "HLA-DR antigen expression in primary melanomas of the skin", J Invest Dermatol 82:244-247 (1984); Becker et al., "Tumor escape mechanisms from immunosurveillance: induction of unresponsiveness in a specific MHC-restricted CD4+ human T cell clone by the autologous MHC class II+ melanoma", Internat Immunol 5:1501-1508 (1993)). As was described in melanomas, head and neck cancers lack the expression of the costimulatory molecules B7.1 and B7.2, and therefore, the expression of MHC class II molecules on head and neck (OSCC) tumor cells may induce immunological tolerance (Lang et al., "Impairment of T-cell activation in head and neck cancer in situ and in vitro", Arch Otolaryngol Head Neck Surg 125:82-88(1999)).

Exclusive targeting of the immune system without a concurrent attempt to modulate the sensitivity of the cancer cell target may not yield an improvement of the overall response rate to an immunologically based treatment.

The immune-mediated processes induced by Leukocyte Interleukin, Injection (LI) or Multikine® may continue long after the cessation of LI administration as evidenced by the immunohistopathological changes observed in the tumor 14-54 days following the end of LI treatment. The efficacy of this neoadjuvant treatment protocol for patients with advanced primary OSCC resulted in a marked reduction in tumor mass in 42% of the patients as compared to baseline tumor measurements. LI neoadjuvant immunotherapy regiment of the OSCC is a clinically viable approach to the management of OSCC.

## Claims

1. A use of serum-free and mitogen-free cytokine mixture comprised of specific ratios of cytokines selected from the group of IL-1β, TNF-α, IFN-γ, GM-CSF, Interleukin-2 (IL-2) in the preparation of a medicament for the treatment of cancer treatable by modulating HLA Class II tumor cell surface expression where the specific ratios of IL-1β, TNF-α, IFN-γ, GM-CSF to IL-2 are:
IL-1β to IL-2 at a ratio range of 0.4 -1.5 ;
TNF-α to IL-2 at a ratio range of 3.2 - 11.3;
IFN-γ to IL-2 at a ratio range of 1.5-10.9; and
GM-CSF to IL-2 at a ratio range of 2.2 - 4.8;
wherein dosage amount of said serum-free and mitogen-free cytokine mixture is five times a week over a three week period in a daily dose of 200, 400, 800, 1200, 1600, 2400, 3200, 4800, 8000, 9600 or 12000 IU, wherein IU represent International Units for Interleukin-2 given in World Health Organization 1^{st} International Standard for Human IL-2, 86/504, and wherein one half of the daily dose is administered peritumorally and the other half of the daily dose is administered perilymphatically, wherein the cancer is oral squamous cell carcinoma and is negative for HLA class II expression,
and wherein the cytokine mixture is administered prior to surgery followed by radiotherapy.

2. The use of claim 1, wherein the daily dosage amount of said serum-free and mitogen free cytokine mixture is 200 IU wherein IU represent International Units for Interleukin-2 given in World Health Organization 1^{st} International Standard for Human IL-2, 86/504.

3. The use of claim 1, wherein the daily dosage amount of said serum-free and mitogen free cytokine mixture is 400 IU wherein IU represent International Units for Interleukin-2 given in World Health Organization 1^{st} International Standard for Human IL-2, 86/504.

4. The use of claim 1, wherein the daily dosage amount of said serum-free and mitogen free cytokine mixture is 800 IU wherein IU represent International Units for Interleukin-2 given in World Health Organization 1^{st} International Standard for Human IL-2, 86/504.

5. The use of claim 1, wherein the daily dosage amount of said serum-free and mitogen free cytokine mixture is 1200 IU wherein IU represent International Units for Interleukin-2 given in World Health Organization 1^{st} International Standard for Human IL-2, 86/504.

6. The use of claim 1, wherein the daily dosage amount of said serum-free and mitogen free cytokine mixture is 1600 IU wherein IU represent International Units for Interleukin-2 given in World Health Organization 1^{st} International Standard for Human IL-2, 86/504.

7. The use of claim 1, wherein the daily dosage amount of said serum-free and mitogen free cytokine mixture is 2400 IU wherein IU represent International Units for Interleukin-2 given in World Health Organization 1^{st} International Standard for Human IL-2, 86/504.

8. The use of claim 1, wherein the daily dosage amount of said serum-free and mitogen free cytokine mixture is 3200 IU wherein IU represent International Units for Interleukin-2 given in World Health Organization 1^{st} International Standard for Human IL-2, 86/504

9. The use of claim 1, wherein the daily dosage amount of said serum-free and mitogen-free cytokine mixture is 4800 IU wherein IU represent International Units for Interleukin-2 given in World Health Organization 1^{st} International Standard for Human IL-2, 86/504.

10. The use of claim 1, wherein the daily dosage amount of said serum-free and mitogen free cytokine mixture is 8000 IU, 9600 IU or 12000 IU wherein IU represent International Units for Interleukin-2 given in World Health Organization 1^{st} International Standard for Human IL-2, 86/504.

11. The use of any of claims 1-10, wherein the serum-free and mitogen free cytokine mixture administered peritumorally is injected into at least four different sites around the mass of a tumor.

12. The use of any of claims 1-11, further comprising an IL-3 to IL-2 ratio in a range from 0.38 - 0.68, preferably at 0.53+/-0.15.

13. The use of any of claims 1-11, further comprising an IL-6 to IL-2 ratio in a range from 37.2 - 53.8, preferably at 46+/-5.9.

14. The use of any of claims 1-11, further comprising an IL-8 to IL-2 ratio in a range from 261 - 561.5, preferably at 411+/-10.6.

15. The use of any of claims 1-11, further comprising an IL-1α to IL-2 ratio in a range from 0.56 - 0.94, preferably at 0.75+/-0.19.

16. The use of any of claims 1-11, further comprising an IL-10 to IL-2 ratio in a range from 2.87 - 3.22, preferably at 3.0+/-0.18.

17. The use of any of claims 1-11, further comprising an IL-16 to IL-2 ratio in a range from 1.16 - 2.84, preferably at 1.84+/-0.68.

18. The use of any of claims 1-11, further comprising a G-CSF to IL-2 ratio in a range from 2.16 - 3.78, preferably at 2.97+/-0.81.

19. The use of any of claims 1-11, further comprising a TNF-β to IL-2 ratio in a range from 1.17 - 2.43, preferably at 1.8+/-0.63.

20. The use of any of claims 1-11, further comprising a MIP-1α to IL-2 ratio in a range from 15.7 - 37.16, preferably at 22.7+/-7.0.

21. The use of any of claims 1-11, further comprising a MIP-1β to IL-2 ratio in a range from 17.1 - 28.5, preferably at 22.8+/-5.7.

22. The use of any of claims 1-11, further comprising a RANTES to IL-2 ratio in a range from 2.3 - 2.7, preferably at 2.5+/-0.13.

23. The use of any of claims 1-11, further comprising an EGF to IL-2 ratio in a range from 0.267 - 0.283, preferably at 0.275+/-0.008.

24. The use of any of claims 1-11, further comprising a PGE₂ to IL-2 ratio in a range from 3.63 - 5.42, preferably at 4.5+/-0.87.

25. The use of any of claims 1-11, further comprising a TxB₂ to IL-2 ratio in a range from 23.47 - 25.13, preferably at 24.3+/-0.83.

26. The use of any of claims 1-11, wherein IL-12 is present in trace quantities.

27. The use of claim 26 wherein trace quantities is defined as 100 pg/mL or less.

## Patentansprüche

1. Verwendung einer serumfreien und mitogenfreien Cytokinmischung, bestehend aus spezifischen Verhältnissen von Cytokinen, ausgewählt aus der Gruppe bestehend aus IL-1β, TNF-α, IFN-γ, GM-CSF, Interleukin-2 (IL-2), bei der Herstellung eines Medikaments zur Behandlung von Krebs, behandelbar durch Modulieren von HLA Klasse II Tumorzelloberflächenexpression, wobei die spezifischen Verhältnisse von IL-1β, TNF-α, IFN-γ, GM-CSF zu IL-2 wie folgt lauten:
IL-1β zu IL-2 in einem Verhältnisbereich von 0,4-1,5;
TNF-α zu IL-2 in einem Verhältnisbereich von 3,2 - 11,3;
IFN-γ zu IL-2 in einem Verhältnisbereich von 1,5 - 10,9; und
GM-CSF zu IL-2 in einem Verhältnisbereich von 2,2 - 4,8;
wobei die Dosismenge der genannten serumfreien und mitogenfreien Cytokinmischung fünfmal die Woche über eine dreiwöchentliche Periode mit einer Tagesdosis von 200, 400, 800, 1200, 1600, 2400, 3200, 4800, 8000, 9600 oder 12000 IU beträgt, wobei IU für internationale Einheiten für Interleukin-2 steht, angegeben im 1^{st} International Standard for Human IL-2, 86/504 der Weltgesundheitsorganisation, und wobei die Hälfte der Tagesdosis peritumoral verabreicht wird und die andere Hälfte der Tagesdosis perilymphatisch verabreicht wird, wobei der Krebs orales Plattenepithelkarzinom ist und für HLA Klasse II-expression negativ ist,
und wobei die Cytokinmischung vor einem chirurgischen Eingriff verabreicht wird, gefolgt von Radiotherapie.

2. Verwendung nach Anspruch 1, wobei die tägliche Dosiermenge der genannten serumfreien und mitogenfreien Cytokinmischung 200 IU beträgt, wobei IU für internationale Einheiten für Interleukin-2 steht, angegeben im 1^{st} International Standard for Human IL-2, 86/504 der Weltgesundheitsorganisation.

3. Verwendung nach Anspruch 1, wobei die tägliche Dosiermenge der genannten serumfreien und mitogenfreien Cytokinmischung 400 IU beträgt, wobei IU für internationale Einheiten für Interleukin-2 steht, angegeben im 1^{st} International Standard for Human IL-2, 86/504 der Weltgesundheitsorganisation.

4. Verwendung nach Anspruch 1, wobei die tägliche Dosiermenge der genannten serumfreien und mitogenfreien Cytokinmischung 800 IU beträgt, wobei IU für internationale Einheiten für Interleukin-2 steht, angegeben im 1^{st} International Standard for Human IL-2, 86/504 der Weltgesundheitsorganisation.

5. Verwendung nach Anspruch 1, wobei die tägliche Dosiermenge der genannten serumfreien und mitogenfreien Cytokinmischung 1200 IU beträgt, wobei IU für internationale Einheiten für Interleukin-2 steht, angegeben im 1^{st} International Standard for Human IL-2, 86/504 der Weltgesundheitsorganisation.

6. Verwendung nach Anspruch 1, wobei die tägliche Dosiermenge der genannten serumfreien und mitogenfreien Cytokinmischung 1600 IU beträgt, wobei IU für internationale Einheiten für Interleukin-2 steht, angegeben im 1^{st} International Standard for Human IL-2, 86/504 der Weltgesundheitsorganisation.

7. Verwendung nach Anspruch 1, wobei die tägliche Dosiermenge der genannten serumfreien und mitogenfreien Cytokinmischung 2400 IU beträgt, wobei IU für internationale Einheiten für Interleukin-2 steht, angegeben im 1^{st} International Standard for Human IL-2, 86/504 der Weltgesundheitsorganisation.

8. Verwendung nach Anspruch 1, wobei die tägliche Dosiermenge der genannten serumfreien und mitogenfreien Cytokinmischung 3200 IU beträgt, wobei IU für internationale Einheiten für Interleukin-2 steht, angegeben im 1^{st} International Standard for Human IL-2, 86/504 der Weltgesundheitsorganisation.

9. Verwendung nach Anspruch 1, wobei die tägliche Dosiermenge der genannten serumfreien und mitogenfreien Cytokinmischung 4800 IU beträgt, wobei IU für internationale Einheiten für Interleukin-2 steht, angegeben im 1^{st} International Standard for Human IL-2, 86/504 der Weltgesundheitsorganisation.

10. Verwendung nach Anspruch 1, wobei die tägliche Dosiermenge der genannten serumfreien und mitogenfreien Cytokinmischung 8000 IU, 9600 IU oder 12000 IU beträgt, wobei IU für internationale Einheiten für Interleukin-2 steht, angegeben im 1^{st} International Standard for Human IL-2, 86/504 der Weltgesundheitsorganisation.

11. Verwendung nach einem der Ansprüche 1-10, wobei die serumfreie und mitogenfreie Cytokinmischung peritumoral verabreicht wird und in mindestens vier unterschiedlichen Stellen um die Masse eines Tumors herum injiziert wird.

12. Verwendung nach einem der Ansprüche 1-11, die ferner ein Verhältnis von IL-3 zu IL-2 in einem Bereich von 0,38 - 0,68, vorzugsweise von 0,53+/-0,15 umfasst.

13. Verwendung nach einem der Ansprüche 1-11, die ferner ein Verhältnis von IL-6 zu IL-2 in einem Bereich von 37,2 - 53,8, vorzugsweise von 46+/-5,9 umfasst.

14. Verwendung nach einem der Ansprüche 1-11, die ferner ein Verhältnis von IL-8 zu IL-2 in einem Bereich von 261 - 561,5, vorzugsweise von 411+/-10,6 umfasst.

15. Verwendung nach einem der Ansprüche 1-11, die ferner ein Verhältnis von IL-1α zu IL-2 in einem Bereich von 0,56 - 0,94, vorzugsweise von 0,75+/-0,19 umfasst.

16. Verwendung nach einem der Ansprüche 1-11, die ferner ein Verhältnis von IL-10 zu IL-2 in einem Bereich von 2,87 - 3,22, vorzugsweise von 3,0+/-0,18 umfasst.

17. Verwendung nach einem der Ansprüche 1-11, die ferner ein Verhältnis von IL-16 zu IL-2 in einem Bereich von 1,16 - 2,84, vorzugsweise von 1,84+/-0,68 umfasst.

18. Verwendung nach einem der Ansprüche 1-11, die ferner ein Verhältnis von G-CSF zu IL-2 in einem Bereich von 2,16 - 3,78, vorzugsweise von 2,97+/-0,81 umfasst.

19. Verwendung nach einem der Ansprüche 1-11, die ferner ein Verhältnis von TNF-β zu IL-2 in einem Bereich von 1,17 - 2,43, vorzugsweise von 1,8+/-0,63 umfasst.

20. Verwendung nach einem der Ansprüche 1-11, die ferner ein Verhältnis von MIP-1α zu IL-2 in einem Bereich von 15,7 - 37,16, vorzugsweise von 22,7+/-7,0 umfasst.

21. Verwendung nach einem der Ansprüche 1-11, die ferner ein Verhältnis von MIP-1β zu IL-2 in einem Bereich von 17,1- 28,5, vorzugsweise von 22,8+/-5,7 umfasst.

22. Verwendung nach einem der Ansprüche 1-11, die ferner ein Verhältnis von RANTES zu IL-2 in einem Bereich von 2,3 - 2,7, vorzugsweise von 2,5+/-0,13 umfasst.

23. Verwendung nach einem der Ansprüche 1-11, die ferner ein Verhältnis von EGF zu IL-2 in einem Bereich von 0,267 - 0,283, vorzugsweise von 0,275+/-0,008 umfasst.

24. Verwendung nach einem der Ansprüche 1-11, die ferner ein Verhältnis von PGE₂ zu IL-2 in einem Bereich von 3,63 - 5,42, vorzugsweise von 4,5+/-0,87 umfasst.

25. Verwendung nach einem der Ansprüche 1-11, die ferner ein Verhältnis von TxB₂ zu IL-2 in einem Bereich von 23,47 - 25,13, vorzugsweise von 24,3+/-0,83 umfasst.

26. Verwendung nach einem der Ansprüche 1-11, wobei IL-12 in Spurenmengen vorliegen.

27. Verwendung nach Anspruch 26, wobei Spurenmengen als 100 pg/ml oder weniger definiert sind.

## Revendications

1. Utilisation d'un mélange de cytokines exempt de sérum et exempt de mitogène composé de rapports spécifiques de cytokines choisies dans le groupe comprenant l'IL-1β, le TNF-α, l'IFN-γ, le GM-CSF, l'interleukine-2 (IL-2), dans la préparation d'un médicament destiné au traitement d'un cancer pouvant être traité par la modulation de l'expression du HLA de classe II sur la surface d'une cellule tumorale, les rapports spécifiques de l'IL-1β, du TNF-α, de l'IFN-γ, du GM-CSF par rapport à l'IL-2 étant :
IL-1β par rapport à IL-2 dans une plage de rapports allant de 0,4 à 1,5 ;
TNF-α par rapport à IL-2 dans une plage de rapports allant de 3,2 à 11,3 ;
IFN-γ par rapport à IL-2 dans une plage de rapports allant de 1,5 à 10,9 ; et
GM-CSF par rapport à IL-2 dans une plage de rapports allant de 2,2 à 4,8 ;
dans laquelle la posologie dudit mélange de cytokines exempt de sérum et exempt de mitogène est cinq fois par semaine sur une période de trois semaines à une dose quotidienne de 200, 400, 800, 1200, 1600, 2400, 3200, 4800, 8000, 9600 ou 12 000 UI, dans laquelle UI représente les unités internationales pour l'interleukine-2 données dans la 1^{ère} norme internationale de l'Organisation mondiale de la santé pour l'IL-2 humaine, 86/504, et dans laquelle la moitié de la dose quotidienne est administrée de manière péri-tumorale et l'autre moitié de la dose quotidienne est administrée de manière péri-lymphatique, dans laquelle le cancer est un cancer épidermoïde buccal et est négatif pour l'expression du HLA de classe II,
et dans laquelle le mélange de cytokines est administré avant une intervention chirurgicale suivie d'une radiothérapie.

2. Utilisation selon la revendication 1, dans laquelle la posologie quotidienne dudit mélange de cytokines exempt de sérum et exempt de mitogène est de 200 UI dans laquelle UI représente les unités internationales pour l'interleukine-2 données dans la 1^{ère} norme internationale de l'Organisation mondiale de la santé pour l'IL-2 humaine, 86/504.

3. Utilisation selon la revendication 1, dans laquelle la posologie quotidienne dudit mélange de cytokines exempt de sérum et exempt de mitogène est de 400 UI dans laquelle UI représente les unités internationales pour l'interleukine-2 données dans la 1^{ère} norme internationale de l'Organisation mondiale de la santé pour l'IL-2 humaine, 86/504.

4. Utilisation selon la revendication 1, dans laquelle la posologie quotidienne dudit mélange de cytokines exempt de sérum et exempt de mitogène est de 800 UI dans laquelle UI représente les unités internationales pour l'interleukine-2 données dans la 1^{ère} norme internationale de l'Organisation mondiale de la santé pour l'IL-2 humaine, 86/504.

5. Utilisation selon la revendication 1, dans laquelle la posologie quotidienne dudit mélange de cytokines exempt de sérum et exempt de mitogène est de 1200 UI dans laquelle UI représente les unités internationales pour l'interleukine-2 données dans la 1^{ère} norme internationale de l'Organisation mondiale de la santé pour l'IL-2 humaine, 86/504.

6. Utilisation selon la revendication 1, dans laquelle la posologie quotidienne dudit mélange de cytokines exempt de sérum et exempt de mitogène est de 1600 UI dans laquelle UI représente les unités internationales pour l'interleukine-2 données dans la 1^{ère} norme internationale de l'Organisation mondiale de la santé pour l'IL-2 humaine, 86/504.

7. Utilisation selon la revendication 1, dans laquelle la posologie quotidienne dudit mélange de cytokines exempt de sérum et exempt de mitogène est de 2400 UI dans laquelle UI représente les unités internationales pour l'interleukine-2 données dans la 1^{ère} norme internationale de l'Organisation mondiale de la santé pour l'IL-2 humaine, 86/504.

8. Utilisation selon la revendication 1, dans laquelle la posologie quotidienne dudit mélange de cytokines exempt de sérum et exempt de mitogène est de 3200 UI dans laquelle UI représente les unités internationales pour l'interleukine-2 données dans la 1^{ère} norme internationale de l'Organisation mondiale de la santé pour l'IL-2 humaine, 86/504.

9. Utilisation selon la revendication 1, dans laquelle la posologie quotidienne dudit mélange de cytokines exempt de sérum et exempt de mitogène est de 4800 UI dans laquelle UI représente les unités internationales pour l'interleukine-2 données dans la 1^{ère} norme internationale de l'Organisation mondiale de la santé pour l'IL-2 humaine, 86/504.

10. Utilisation selon la revendication 1, dans laquelle la posologie quotidienne dudit mélange de cytokines exempt de sérum et exempt de mitogène est de 8000 UI, 9600 UI ou 12 000 UI dans laquelle UI représente les unités internationales pour l'interleukine-2 données dans la 1^{ère} norme internationale de l'Organisation mondiale de la santé pour l'IL-2 humaine, 86/504.

11. Utilisation selon l'une quelconque des revendications 1 - 10, dans laquelle le mélange de cytokines exempt de sérum et exempt de mitogène administré de manière péri-tumorale est injecté dans au moins quatre sites différents autour de la masse d'une tumeur.

12. Utilisation selon l'une quelconque des revendications 1 - 11, comprenant en outre un rapport IL-3 sur IL-2 situé dans une plage allant de 0,38 à 0,68, de préférence à 0,53 +/- 0,15.

13. Utilisation selon l'une quelconque des revendications 1 - 11, comprenant en outre un rapport IL-6 sur IL-2 situé dans une plage allant de 37,2 à 53,8, de préférence à 46 +/-5,9.

14. Utilisation selon l'une quelconque des revendications 1 - 11, comprenant en outre un rapport IL-8 sur IL-2 situé dans une plage allant de 261 à 561,5, de préférence à 411 +/- 10,6.

15. Utilisation selon l'une quelconque des revendications 1 - 11, comprenant en outre un rapport IL-1α sur IL-2 situé dans une plage allant de 0,56 à 0,94, de préférence à 0,75 +/- 0,19.

16. Utilisation selon l'une quelconque des revendications 1 - 11, comprenant en outre un rapport IL-10 sur IL-2 situé dans une plage allant de 2,87 à 3,22, de préférence à 3, 0 +/- 0,18.

17. Utilisation selon l'une quelconque des revendications 1 - 11, comprenant en outre un rapport IL-16 sur IL-2 situé dans une plage allant de 1,16 à 2,84, de préférence à 1,84 +/- 0,68.

18. Utilisation selon l'une quelconque des revendications 1 à 11, comprenant en outre un rapport G-CSF sur IL-2 situé dans une plage allant de 2,16 à 3,78, de préférence à 2,97 +/- 0,81.

19. Utilisation selon l'une quelconque des revendications 1 à 11, comprenant en outre un rapport TNF-β sur IL-2 situé dans une plage allant de 1,17 à 2,43, de préférence à 1,8 +/- 0,63.

20. Utilisation selon l'une quelconque des revendications 1 à 11, comprenant en outre un rapport MIP-1α sur IL-2 situé dans une plage allant de 15,7 à 37,16, de préférence à 22,7 +/- 7,0.

21. Utilisation selon l'une quelconque des revendications 1 à 11, comprenant en outre un rapport MIP-1β sur IL-2 situé dans une plage allant de 17,1 à 28,5, de préférence à 22,8 +/- 5,7.

22. Utilisation selon l'une quelconque des revendications 1 - 11, comprenant en outre un rapport RANTES sur IL-2 situé dans une plage allant de 2,3 à 2,7, de préférence à 2,5 +/- 0,13.

23. Utilisation selon l'une quelconque des revendications 1 -11, comprenant en outre un rapport EGF sur IL-2 situé dans une plage allant de 0,267 à 0,283, de préférence à 0,275 +/- 0,008.

24. Utilisation selon l'une quelconque des revendications 1 à 11, comprenant en outre un rapport PGE₂ sur IL-2 situé dans une plage allant de 3,63 à 5,42, de préférence à 4,5 +/- 0,87.

25. Utilisation selon l'une quelconque des revendications 1 - 11, comprenant en outre un rapport TxB₂ sur IL-2 situé dans une plage allant de 23,47 à 25,13, de préférence à 24,3 +/- 0,83.

26. Utilisation selon l'une quelconque des revendications 1 - 11, dans laquelle l'IL-12 est présente en quantité infime.

27. Utilisation selon la revendication 26, dans laquelle la quantité infime est définie comme étant 100 pg/ml ou moins.
